# EUROPEAN PATENT APPLICATION

(11) **EP 4 722 187 A1**
(43) Date of publication of application: **08.04.2026**
(21) Application number: 24811417.5
(22) Date of filing: 23.05.2024
(51) Int. Cl.: C07B 59/00, C07C 53/18, C07C 211/29, C07F 1/08, A61K 51/04, G01N 33/60, A61K 49/06

(54) **BUILDING BLOCK FOR TRIFLUOROALKYLATION AND TRIFLUOROALKYLATION METHOD USING SAME**

(30) Priority: 24.05.2023 KR 20230067268; 09.05.2024 KR 20240061446
(71) Applicant: Seoul National University Hospital, Seoul 03080 (KR)
(72) Inventor: LEE, Byung Chul, Hanam-si Gyeonggi-do 13014 (KR); CHOI, Ji Young, Seoul 05117 (KR)
(74) Representative: Müller, Christian Stefan Gerd
(86) International application number: PCT/KR2024/006984
(87) International publication number: WO 2024/242479

(57) **Abstract**

The present invention relates to: a building block which can be used for trifluoroalkylation in preparing a radioactive fluorine-18-labeled radiopharmaceutical for medical diagnosis; and a trifluoroalkylation method using same.

## Description

### Technical Field

The present invention relates to a building block that may be used for trifluoroalkylation in the production of a radioactive fluorine-18-labeled radiopharmaceutical for medical diagnosis, a production method thereof, and a method of trifluoroalkylating a ligand using the same.

Meanwhile, this application was supported by the following national research and development project.
[National Research and Development Project that supported this invention]
[Project Serial Number] 1711191663
[Project Number] 2022R1F1A1072205
[Government Department] Ministry of Science and ICT
[Project Management (Specialized) Agency] National Research Foundation of Korea
[Research Project Name] Basic Research
[Research Task Name] Development of new CF2[18F]-labeling technology for rapid and accurate evaluation of new drug
[Agency Carrying Out Project] Seoul National University Bundang Hospital
[Research Period] June 1, 2022 to February 28, 2025
[National Research and Development Project that supported this invention]
[Project Serial Number] 1711179759
[Project Number] 2021R1A2C2003301
[Government Department] Ministry of Science and ICT
[Project Management (Specialized) Agency] National Research Foundation of Korea
[Research Project Name] Mid-Sized Research
[Research Task Name] Development of pH- and temperature-sensitive nanocomposite-based nanocarriers that enhance the loading capacity, tumor selectivity, and retention of mitochondria-targeted tumor therapeutic drugs
[Agency Carrying Out Project] Seoul National University Bundang Hospital
[Research Period] March 1, 2021 to February 8, 2026

### Background Art

Positron Emission Tomography (PET) is an imaging technique primarily used in nuclear medicine. It noninvasively demonstrates the distribution and activity of radiopharmaceuticals injected into the body, providing information on disease diagnosis and treatment effects. Fluorine-18 is the most widely used radioisotope in PET due to its suitable half-life of 110 minutes and its physical and chemical properties. Methods for producing radiopharmaceuticals labeled with fluorine-18 may be divided into two methods. One is a method of labeling a compound of interest having biological activity with fluorine-18 in the last step of the reaction (late-stage radiofluorination), and the other is a method of effectively introducing fluorine-18 into a compound of interest using a building block already labeled with fluorine-18.

The method of labeling with fluorine-18 in the last step has been used to overcome the limitations of the fluorine-18 labeling reaction over the past 10 years, but limitations such as instability of the precursor, complexity of synthesis, and time consumption in the separation and purification process still remain. For this reason, the method of using the building block has been studied as an alternative method for fluorine-18 labeling reaction, as it has the advantage of being available for purchase and easy to synthesize. Furthermore, the development of a building block labeled with fluorine-18 with a high labeling yield and purity through a simple method under appropriate conditions has a great advantage in terms of expandability of use, as fluorine-18 may be subsequently introduced into various biologically active molecules using this building block.

Until recently, in the production of radiopharmaceuticals, [¹⁸F]fluoropropylsulfonyl chloride reagents and the like have been known as building blocks used in the [¹⁸F]fluoroethyl group substitution reaction. However, when these reagents are used, a ligand is labeled with a -CH₂[¹⁸F] group, but this structure has a problem in that the *in vivo* stability or cell penetration ability thereof is not high.

The inventors of the present invention have made extensive efforts, and as a result, have developed a building block capable of labeling a ligand with a -CF₂[¹⁸F] group to improve the *in vivo* stability or cell penetration ability of a radiopharmaceutical and a method for producing the same, thereby reaching the present invention.

### DISCLOSURE

### Technical Problem

An object of the present invention is to provide a building block that may be used for trifluoroalkylation and a method for producing the same.

Another object of the present invention is to provide a method of trifluoroalkylating a ligand using the above-described building block.

Another object of the present invention is to provide a radiopharmaceutical labeled with fluorine-18 by substitution with a -CF₂[¹⁸F] group through the above-described building block.

However, the technical problems to be solved by the present invention are not limited to the problems mentioned above, and other problems not mentioned may be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

The term "halogen" as used herein, unless otherwise stated, means fluorine, chlorine, bromine, or iodine.

The term "C₁-C₆ alkyl" as used herein, unless otherwise stated, means a straight-chain or branched hydrocarbon residue having 1 to 6 carbon atoms. Examples thereof include, but are not limited to, methyl, ethyl, propyl, isopropyl, n-butyl, sec-butyl, t-butyl, n-pentyl, n-hexyl, and the like.

The term "C₁-C₆ alkoxy" as used herein means -O-alkyl, wherein the alkyl is as disclosed above. Examples thereof include methoxy, ethoxy, n-propoxy, isopropoxy, n-butoxy, t-butoxy, sec-butoxy, n-pentoxy, etc. The alkoxy may be substituted or unsubstituted alkoxy.

The term "heterocycle" as used herein is understood to mean a partially unsaturated (heterocycloalkenyl) hydrocarbon ring having 3 to 15 carbon atoms in the form of a monocyclic, bicyclic, fused, bridged or spirocyclic ring.

The term "aryl" as used herein, unless otherwise stated, refers to a mono- or polycyclic carbocyclic ring system of 6 to 14 carbon atoms having one or more aromatic rings, fused or non-fused, and examples of aryl include, but are not limited to, phenyl, naphthyl, tetrahydronaphthyl, indenyl, and andracenyl.

The term "heteroaryl" as used herein, unless otherwise stated, means a 5- to 14 membered monocyclic or bicyclic or higher aromatic group containing one or more, for example, 1 to 4 heteroatoms heteroatoms, selected from O, N and S. Examples of monocyclic heteroaryls include, but are not limited to, thiazolyl, oxazolyl, thiophenyl, furanyl, pyrrolyl, imidazolyl, benzo[d]oxazolyl, isoxazolyl, oxazolopyridinyl, pyrazolyl, triazolyl, thiazolyl, benzo[d]thiazolyl, thiadiazolyl, tetrazolyl, oxadiazolyl, pyridinyl, pyridazinyl, pyrimidinyl, pyrazinyl, quinolinyl, naphthooxazolyl, and groups similar thereto. Examples of bicyclic heteroaryls include, but are not limited to, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, purinyl, furopyridinyl, carbazolyl, dibenzofuranyl, dibenzothiophenyl, and groups similar thereto.

According to one embodiment of the present invention, the present invention relates to a building block composition for trifluoroalkylation comprising a copper complex compound represented by Formula 1 below.

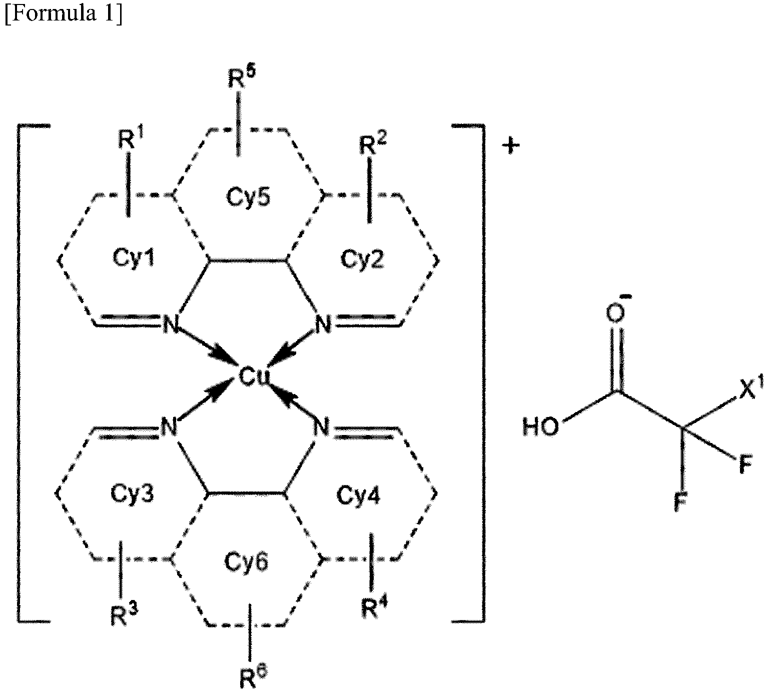

In Formula 1 above,
Cy1 to Cy4 are each independently a 4- to 8-membered heteroaryl or heterocycloalkenyl ring,
Cy5 and Cy6 are each independently a 0- to 8-membered cycloalkyl, cycloalkenyl, aryl, heteroaryl, heterocycloalkyl or heterocycloalkenyl ring (wherein 0-membered means there is no ring),
R¹ to R⁶ are each independently hydrogen, a halogen, nitro, a C₁-C₆ alkyl group, or a C₆-C₁₄ aryl group, and are mono-substituents or the same or different multi-substituents, and
X¹ may be a halogen other than a fluoro group.

In the present invention, the copper complex compound represented by Formula 1 may be used as a building block for trifluoroalkylation or fluorine-18 labeled trifluoroalkylation substitution of a target ligand.

In the present invention, R¹ to R⁶ may each independently be hydrogen or a C₁-C₆ alkyl group, without being limited thereto.

In the present invention, X¹ may be a chloro group, a bromo group, or an iodo group, without being limited thereto.

In the present invention, the copper complex compound represented by Formula 1 may be a compound represented by Formula 6 or 7 below, without being limited thereto.

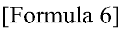

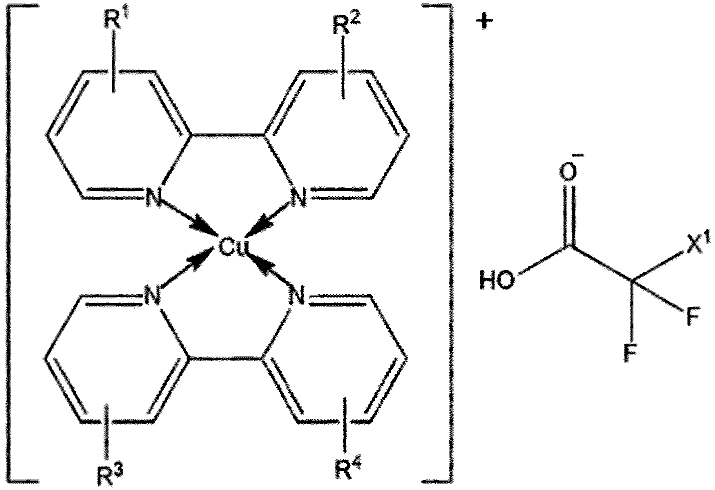

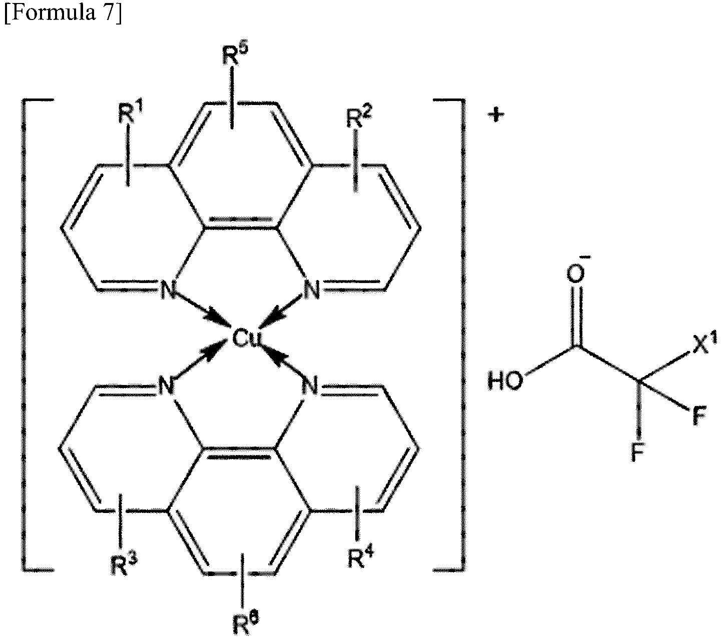

In Formulas 6 and 7 above, R¹ to R⁶ and X¹ are as defined above.

In the present invention, the copper complex compound represented by Formula 1 may be a compound represented by any one of Formulas 11 to 13 below, without being limited thereto.

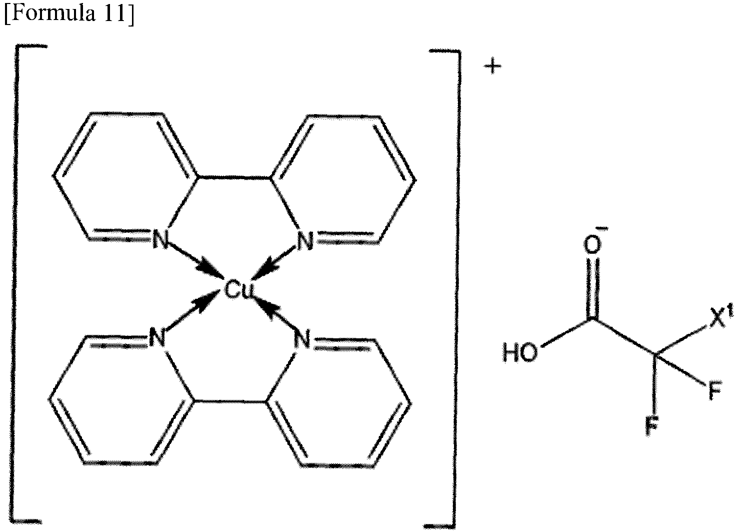

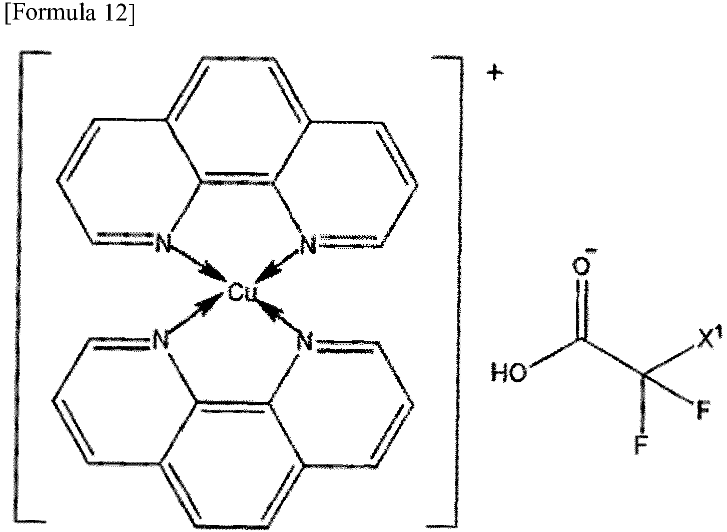

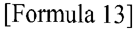

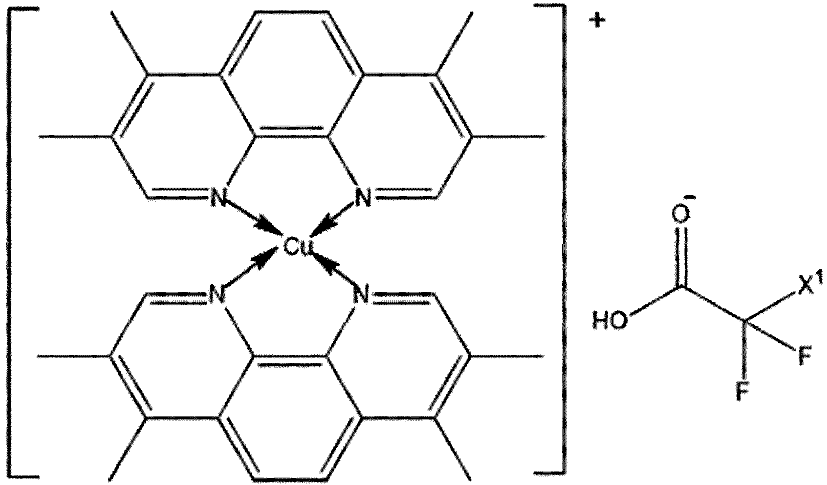

In Formulas 11 to 13 above, X¹ is as defined above.

According to another embodiment of the present invention, the present invention relates to a method for producing the copper complex compound represented by Formula 1.

In the production method of the present invention, the copper complex compound of Formula 1 may be produced by reacting a compound represented by Formula 2 below with a copper complex represented by Formula 3 below:

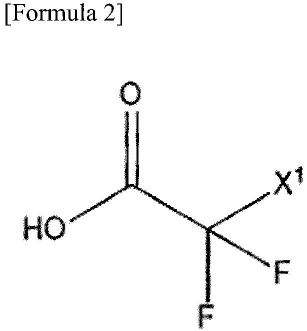

In Formula 2 above, X¹ is as defined above.

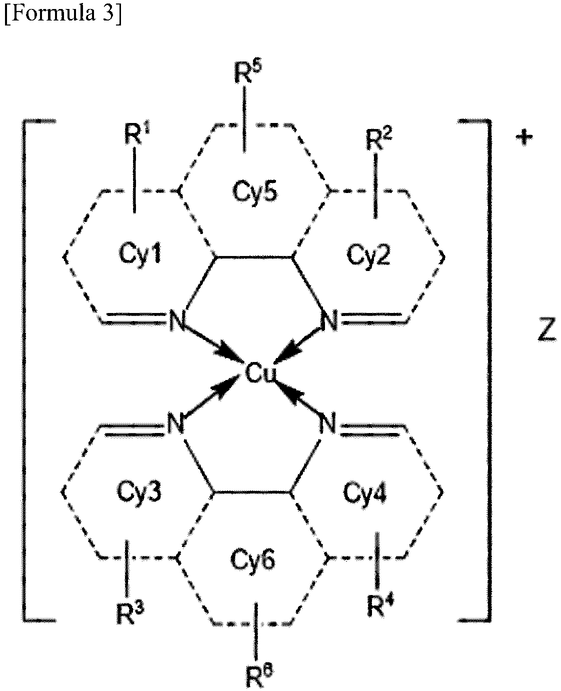

In Formula 3,
Z is a counterion, for example, NO₃⁻, CO₃⁻, ClO₄⁻, I⁻, SCN⁻ or BF₄⁻, and
Cy1 to Cy6 and R¹ to R⁶ are each as defined above.

In the present invention, the copper complex represented by Formula 3 may be a compound represented by Formula 4 or 5 below, without being limited thereto.

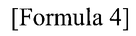

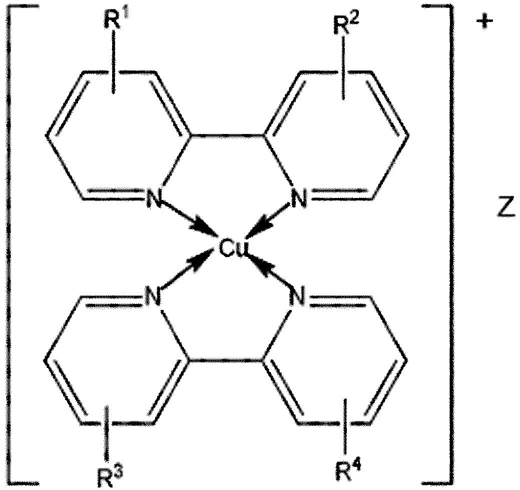

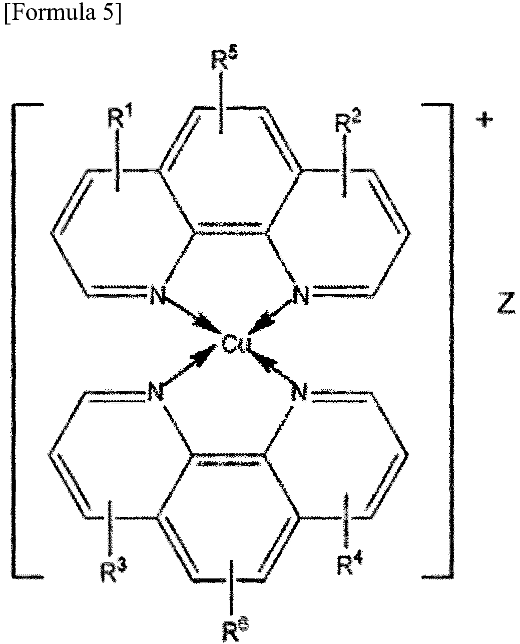

In Formulas 4 and 5 above, R¹ to R⁶ and Z are as defined above.

In the present invention, a copper complex compound represented by Formula 6 or 7 below may be produced by reacting the compound represented by Formula 2 with the copper complex represented by Formula 4 or 5.

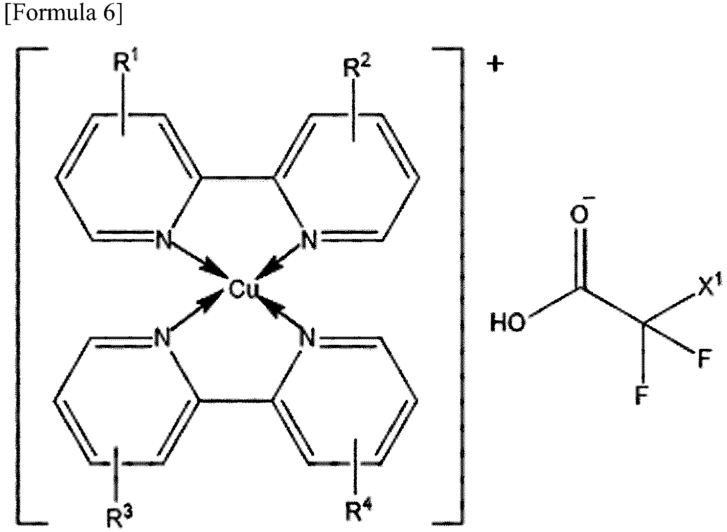

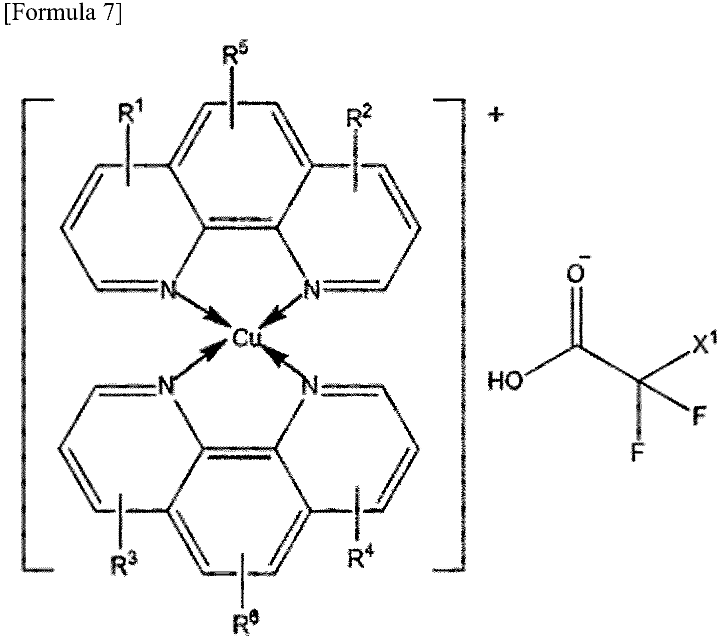

In Formulas 6 and 7 above, R¹ to R⁶ and X¹ are as defined above.

In the present invention, the copper complex represented by Formula 3 may be a compound represented by any one of Formulas 8 to 10 below, without being limited thereto.

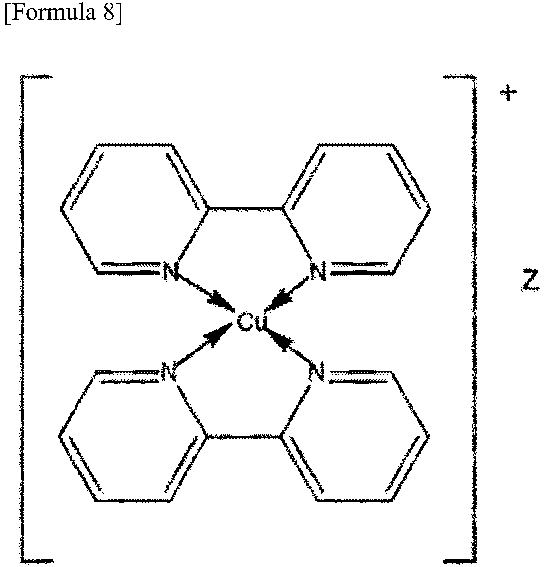

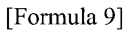

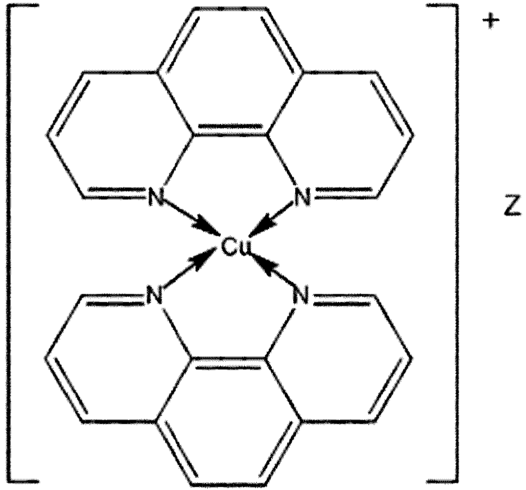

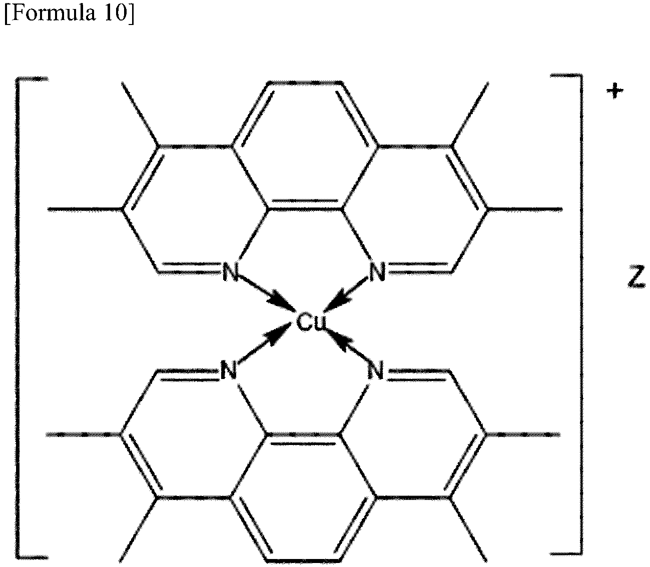

In Formulas 8 to 10 above, Z is as defined above.

In the present invention, a copper complex compound represented by any one of Formulas 11 to 13 below may be produced by reacting the compound represented by Formula 2 with the copper complex represented by any one of Formulas 8 to 10.

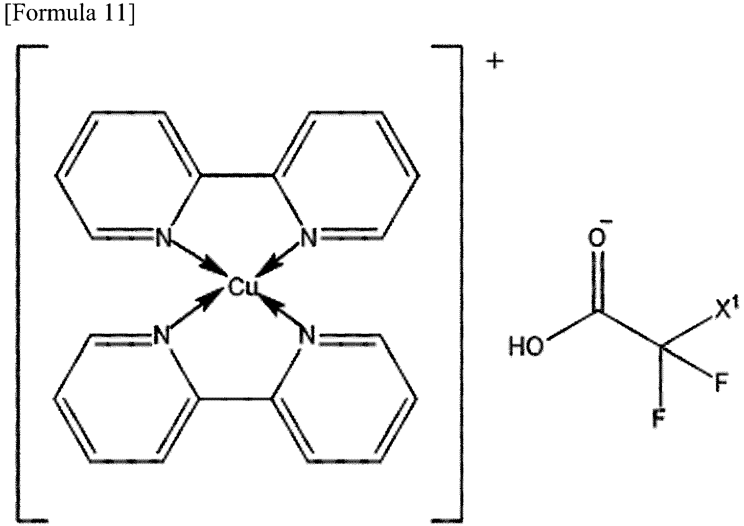

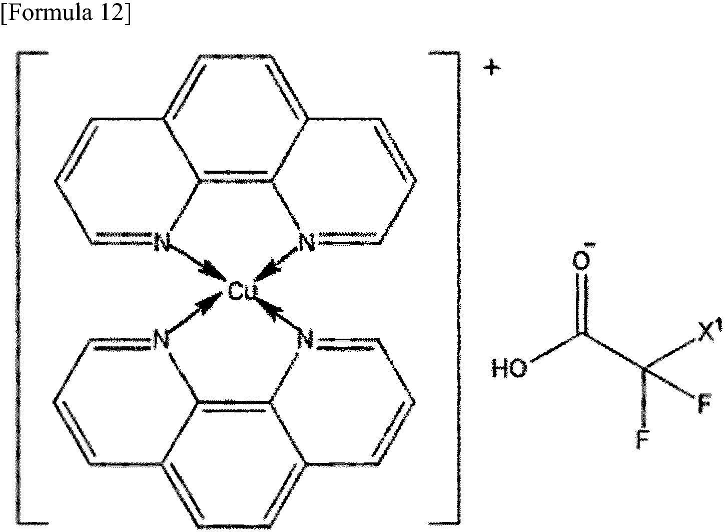

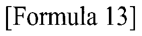

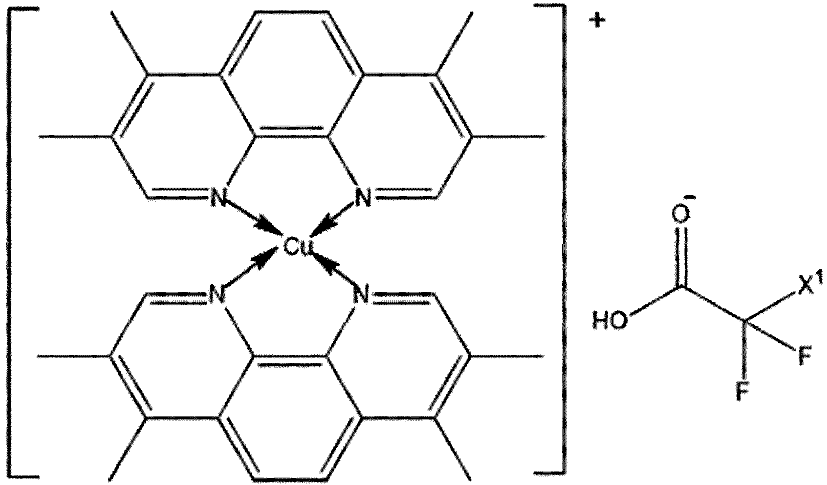

In Formulas 11 to 13 above, X¹ is as defined above.

In the present invention, the copper complex represented by Formula 3 may be produced by reacting a copper source and a ligand for producing a copper complex in the presence of a base. Here, as the copper source, a copper halide such as CuF, CuCl, CuBr or CuI may be used, or Cu(OTf)₂, Cu(py)₄(OTf)₂, Cu(NCCH₃)₄, Cu(NCCH₃)₄·CF₃SO₃₃ Cu(MeCN)₄BF₄, [(CH₃CN)₄Cu]PF₆, or (CF₃SO₃Cu)₂·C₆H₆ may be used. In addition, the ligand for producing a copper complex may be an N-donor ligand or an N,N'-donor ligand, and specific examples thereof include, but are not limited to, pyridine-2-carboxylic acid, dipivaloylmethane, N,N-dimethylglycine, 1,10-phenanthroline, proline, 2-thiophenecarboxylic acid, 2,2'-bipyridyl, ethyl 2-oxocyclohexanecarboxylate, 3,4,7,8-tetramethyl-1,10-phenanthroline, and N,N-dimethylethylenediamine. In the present invention, the ligand for producing a copper complex may be added in an amount of 1 to 3 equivalents relative to the copper source, without being limited thereto.

In addition, in the present invention, the reaction for producing the copper complex may be carried out in the presence of a base. Here, as the base to be added, alkoxides, such as sodium tert-butoxide; alkali metal amides, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 2 equivalents, preferably 1 to 1.5 equivalents, relative to the copper source, without being limited thereto.

In the present invention, the reaction of the compound of Formula 2 with the copper complex may be performed in a solvent. Here, as the solvent, any one or more selected from the group consisting of CH₃CN, tetrahydrofuran (THF), CH₂Cl₂, N,N-dimethylformamide (DMF), dimethyl sulfoxide (DMSO), and mixtures thereof may be used, without being limited thereto.

In the present invention, the reaction of the compound of Formula 2 with the copper complex may be performed at room temperature (25±5°C) for 1 minute to 1 hour, preferably 5 to 30 minutes, without being limited thereto.

In the present invention, to react the compound of Formula 2 with the copper complex, the copper complex may be added in an amount of 0.5 to 2 equivalents, preferably 0.75 to 1.5 equivalents, relative to the compound, without being limited thereto.

In the present invention, the reaction for producing the copper complex and the reaction of the copper complex with the compound of Formula 2 may be performed sequentially, but may also be performed simultaneously. Furthermore, they may be performed as a one-pot reaction, without being limited thereto.

According to another embodiment of the present invention, the present invention relates to a method of producing a trifluoroacetic acid represented by Formula 14 below by reacting the compound represented by Formula 2 or the copper complex compound represented by Formula 1 with [¹⁸F]fluoride ion or [¹⁹F]fluoride ion:

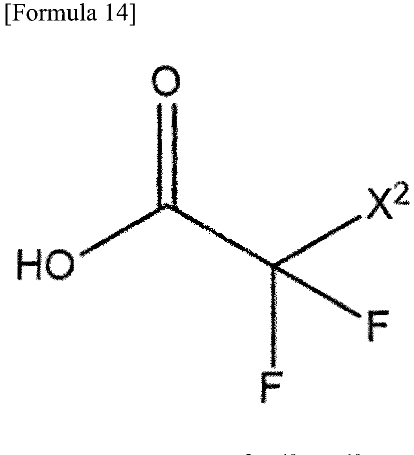

In Formula 14 above, X² is ¹⁸F or ¹⁹F.

In the present invention, the trifluoroacetic acid of Formula 14 may be produced by reacting each of the compound of Formula 2 and the copper complex compound represented by Formula 1 with fluoride ion.

In the present invention, the copper complex compound of Formula 1 may be produced by reacting the compound of Formula 2 with the copper complex represented by Formula 3 as described above.

In the present invention, the [¹⁸F]fluoride ion or [¹⁹F]fluoride ion may be added in an amount of 0.2 to 4 equivalents, preferably 0.2 to 2 equivalents, more preferably 0.2 to 1.5 equivalents, most preferably 1 equivalent, relative to the copper complex compound represented by Formula 1, without being limited thereto.

In the present invention, the reaction of the compound represented by Formula 2 or the copper complex compound represented by Formula 1 with [¹⁸F]fluoride ion or [¹⁹F]fluoride ion is preferably performed in the presence of a base so that the reaction efficiency may be increased. Here, as the base, alkoxides, such as sodium tert-butoxide; an alkali metal amide, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 2 equivalents, preferably 0.75 to 1.5 equivalents, most preferably 1 equivalent, relative to the compound represented by Formula 2 or 4, without being limited thereto.

In the present invention, the reaction of the compound represented by Formula 2 or the copper complex compound of Formula 1 with the fluoride ion may be performed in a solvent. Here, as the solvent, any one or more selected from the group consisting of CH₃CN, tetrahydrofuran (THF), CH₂Cl₂, N,N-dimethylformamide (DMF), dimethyl sulfoxide (DMSO), and mixtures thereof may be used, without being limited thereto.

In addition, in the present invention, the reaction of the compound represented by Formula 2 or the copper complex compound represented by Formula 1 with the fluoride ion may be performed at a temperature of 50 to 100 °C, preferably 60 to 80 °C, more preferably 75 °C, without being limited thereto.

In addition, in the present invention, the reaction of the compound represented by Formula 2 or the copper complex compound represented by Formula 1 with the fluoride ion may be performed for 10 minutes to 6 hours, preferably 10 minutes to 1 hour, more preferably 10 minutes to 30 minutes, without being limited thereto.

In the present invention, the [¹⁸F]fluoride ion may be obtained through the 18O(p,n)¹⁸F reaction from a cyclotron, without being limited thereto. In the present invention, if necessary, a step of obtaining a [¹⁸F]F⁻/H₂¹⁸O solution by separating the [¹⁸F]fluoride obtained through the above reaction using an acetonitrile reaction solution containing K_{2.2.2} and K₂CO₃ dissolved therein, and a step of obtaining K_{2.2.2}/K[¹⁸F]F by heating the [¹⁸F]F⁻/H₂¹⁸O solution may be additionally performed, and the obtained K_{2.2.2}/K[¹⁸F]F may be used.

In addition, in the present invention, the [¹⁸F] fluoride ion may be used after moisture is removed therefrom by adding [¹⁸F] fluoride to a reaction vessel together with a phase transfer catalyst and a base additive under a solvent, and then blowing nitrogen at a temperature of 80 to 100°C. Here, the solvent may be methyl alcohol, acetonitrile, or a mixture thereof. As the phase transfer catalyst, Kryptofix_{2.2.2} (K_{2.2.2}) or 18-crown-6 may be used, and as the basic additive, one selected from the group consisting of CsHCO₃, Cs₂CO₃, KHCO₃, K₂CO₃ and tetrabutylammonium salt may be used, without being limited thereto.

The production method of the present invention may, if necessary, further comprise a step of separating and purifying trifluoroacetic acid from the reaction mixture obtained after the reaction. In the present invention, the separation and purification method is not particularly limited, but may be performed by passing the reaction mixture through a cartridge containing an alumina adsorbent (sorbent substrate), and for example, may be performed by using a Sep-pak^{®} alumina N cartridge. Preferably, in terms of the separation and purification efficiency of the trifluoroacetic acid, the reaction mixture may be passed through the cartridge filled with 250 to 2,000 mg of the alumina adsorbent, and more preferably, may be passed through the cartridge filled with 1,000 to 2,000 mg of the alumina adsorbent. As a specific example, an alumina N light cartridge filled with 280 mg of the alumina adsorbent may be used, or an alumina N plus cartridge filled with 1,710 mg of the alumina adsorbent may be used, and more preferably, an alumina N plus cartridge may be used, without being limited thereto.

In the present invention, the [¹⁹F] fluoride ion may be derived from a metal fluoride compound, without being limited thereto, and any fluoride source capable of supplying [¹⁹F] fluoride ion may be included without limitation. Here, examples of the metal fluoride compound that may be used include, but are not limited to, LiF, NaF, KF, CsF, RbF, TiF, AgF, AgF₂, BaF₂, CaF₂, CuF₂, CdF₂, FeF₂, HgF₂, Hg₂F₂, MnF₂, MgF₂, NiF₂, PbF₂, SnF₂, SrF₂, XeF₂, ZnF₂, AlF₂, BF₂, BiF₂, CeF₂, CrF₃, DyF₃, EuF₃, GaF₃, GdF₃, FeF₃, HoF₃, InF₃, LaF₃, LuF₃, MnF₃, NdF₃, VOF₃, PrF₃, SbF₃, ScF₃, SmF₃, TbF₃, TiF₃, TmF₃, YF₃, YbF₃, TIF₃, CeF₄, GeF₄, HfF₄, SiF₄, SnF₄, TiF₄, VF₄, ZrF₄, NbF₅, SbF₅, TaF₅, BiF₅, MoF₆, ReF₆, SF₆ and WF₆.

The production method of the present invention may, if necessary, further comprise a step of formulating the reaction mixture obtained after the reaction. In the present invention, the formulation method is not particularly limited, but may be performed by passing the reaction mixture through a cartridge containing a copolymer sorbent, and for example, may be performed using an Oasis WAX Plus short cartridge, without being limited thereto.

When the production method of the present invention is used, it is possible to produce trifluoroacetic acid in high yield. In the present invention, trifluoroacetic acid labeled with fluorine-18 may be produced by reacting the compound represented by Formula 2 or the copper complex compound of Formula 1 with [¹⁸F] fluoride ion, and the fluorine-18-labeled trifluoroacetic acid produced as described above may be used for the production of additional radiopharmaceuticals.

According to another embodiment of the present invention, the present invention relates to a method of trifluoroalkylating a ligand using the copper complex compound represented by Formula 1.

The trifluoroalkylation method of the present invention may comprise a step of reacting a ligand with the copper complex compound of Formula 1 in the presence of a fluoride ion, thereby trifluoroalkylating the ligand.

In the present invention, the fluoride ion may be [¹⁸F]fluoride ion or [¹⁹F]fluoride ion depending on the purpose. When [¹⁸F]fluoride ion is used as the fluoride ion, the ligand may be labeled with the radioactive isotope fluorine-18.

In the present invention, the [¹⁸F]fluoride ion may be obtained through the 18O(p,n)¹⁸F reaction from a cyclotron, without being limited thereto. In the present invention, if necessary, a step of obtaining a [¹⁸F]F⁻/H₂¹⁸O solution by separating the [¹⁸F]fluoride obtained through the above reaction using an acetonitrile reaction solution containing K_{2.2.2} and K₂CO₃ dissolved therein, and a step of obtaining K_{2.2.2}/K[¹⁸F]F by heating the [¹⁸F]F⁻/H₂¹⁸O solution may be additionally performed, and the obtained K_{2.2.2}/K[¹⁸F]F may be used.

In addition, in the present invention, the [¹⁸F] fluoride ion may be used after moisture is removed therefrom by adding [¹⁸F] fluoride to a reaction vessel together with a phase transfer catalyst and a base additive under a solvent, and then blowing nitrogen at a temperature of 80 to 100°C. Here, the solvent may be methyl alcohol, acetonitrile, or a mixture thereof. As the phase transfer catalyst, K_{2.2.2} or 18-crown-6 may be used, and as the basic additive, one selected from the group consisting of CsHCO₃, Cs₂CO₃, KHCO₃, K₂CO₃ and tetrabutylammonium salt may be used, without being limited thereto.

In the present invention, the [¹⁹F] fluoride ion may be derived from a metal fluoride compound, without being limited thereto, and any fluoride source capable of supplying [¹⁹F] fluoride ion may be included without limitation. Here, examples of the metal fluoride compound that may be used include, but are not limited to, LiF, NaF, KF, CsF, RbF, TiF, AgF, AgF₂, BaF₂, CaF₂, CuF₂, CdF₂, FeF₂, HgF₂, Hg₂F₂, MnF₂, MgF₂, NiF₂, PbF₂, SnF₂, SrF₂, XeF₂, ZnF₂, AlF₂, BF₂, BiF₂, CeF₂, CrF₃, DyF₃, EuF₃, GaF₃, GdF₃, FeF₃, HoF₃, InF₃, LaF₃, LuF₃, MnF₃, NdF₃, VOF₃, PrF₃, SbF₃, ScF₃, SmF₃, TbF₃, TiF₃, TmF₃, YF₃, YbF₃, TIF₃, CeF₄, GeF₄, HfF₄, SiF₄, SnF₄, TiF₄, VF₄, ZrF₄, NbF₅, SbF₅, TaF₅, BiF₅, MoF₆, ReF₆, SF₆ and WF₆.

In the present invention, the fluoride ion may be added in an amount of 0.2 to 4 equivalents, preferably 0.2 to 3 equivalents, more preferably 1 to 3 equivalents, relative to the copper complex compound represented by Formula 1, without being limited thereto.

In the present invention, the reaction is preferably performed in the presence of a base so that the reaction efficiency may be increased. Here, as the base, alkoxides, such as sodium tert-butoxide; alkali metal amides, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 4 equivalents, preferably 0.5 to 3 equivalents, relative to the copper complex compound of Formula 1, without being limited thereto.

In the present invention, the reaction of the copper complex compound represented by Formula 1 with the fluoride ion may be performed in a solvent. Here, as the solvent, any one or more selected from the group consisting of CH₃CN, tetrahydrofuran (THF), CH₂Cl₂, N,N-dimethylformamide (DMF), dimethyl sulfoxide (DMSO), and mixtures thereof may be used, without being limited thereto.

In the present invention, even if the same copper complex compound of Formula 1 is used, the ligand may be substituted with a trifluoromethyl group or a trifluoroethyl group depending on the ligand and the reaction conditions.

### Trifluoromethylation Method

In the present invention, as the ligand to be substituted with the trifluoromethyl group, any ligand containing an aryl (e.g., C₆-C₁₄ aryl) or heteroaryl (e.g., heteroaryl having 5 to 14 nuclear atoms) structure substituted with at least one substituent selected from among a halogen, a boronic acid group (-B(OH)₂), and a boronic acid pinacol ester group (-Bpin) may be included without limitation as long as it may preferably be used for the purpose of diagnosing or treating a disease or for imaging.

In the present invention, the ligand containing the halogen-substituted aryl or heteroaryl structure may contain a phenyl structure substituted with a fluoro group, a chloro group, a bromo group, an iodo group, a boronic acid group (-B(OH)₂), or a boronic acid pinacol ester group (-Bpin), and may be, for example, a compound represented by Formula 15 below, without being limited thereto.

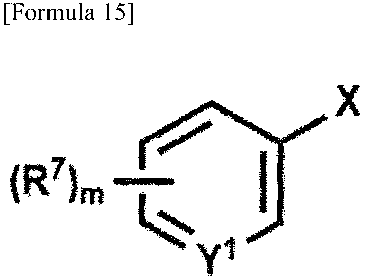

In Formula 15 above,
X is a halogen, a boronic acid group or a boronic acid pinacol ester group, preferably a halogen,
Y¹ is N or C(R⁸),
m is an integer ranging from 0 to 4,
R⁷ is a halogen, nitro, a cyano group, a C₁-C₆ alkyl group, a C₆-C₁₄ aryl group, a C₁-C₆ alkoxy group, -C(=O)-R⁹, or -NR¹⁰C(=O)-R¹¹, and when there are a plurality of R⁷s, they are the same as or different from each other,
R⁸ is hydrogen or a C₁-C₆ alkyl group,
R⁹ is hydrogen, an amine group (-NH₂), a C₁-C₆ alkyl group, or a C₁-C₆ alkoxy group, and
R¹⁰ and R¹¹ are each independently hydrogen or a C₁-C₆ alkyl group.

In the present invention, non-limiting examples of the ligand containing the halogen-substituted aryl or heteroaryl structure include, but are not limited to, compounds having the following structures:

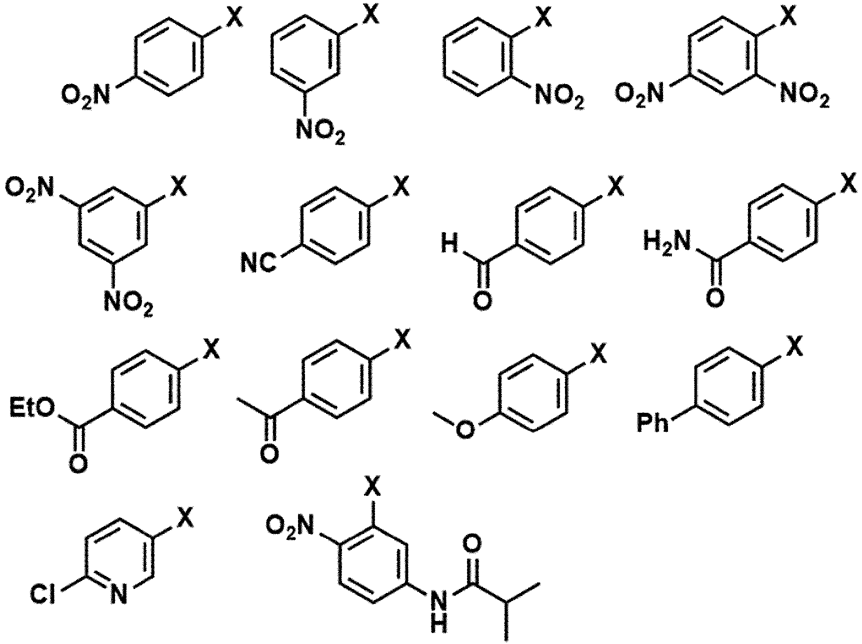

In the above formulas, X is a halogen, a boronic acid group, or a boronic acid pinacol ester group.

The trifluoromethylation method of the present invention may comprise a step of reacting the above-described ligand with the copper complex compound of Formula 1 and a fluoride ion source.

In the present invention, the halogen, boronic acid group or boronic acid pinacol ester group in the ligand may be substituted with a trifluoromethyl group derived from the compound of Formula 1 by a reaction represented by Reaction Scheme 1 below.

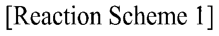

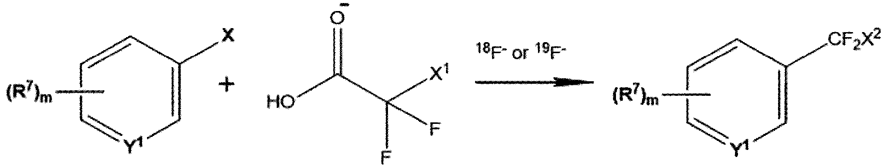

In Reaction Scheme 1 above, each group is as defined above.

In the present invention, the fluoride ion may be added in an amount of 0.2 to 4 equivalents, preferably 1 to 3 equivalents, relative to the copper complex compound represented by Formula 1, without being limited thereto.

In the present invention, the reaction is preferably performed in the presence of a base so that the reaction efficiency may be increased. The base may be added in an amount of 0.5 to 4 equivalents, or 0.5 to 3 equivalents, relative to the copper complex compound of Formula 1. Preferably, when [¹⁸F]fluoride ion is used as the fluoride ion in the reaction, the base may be added in an amount of 0.5 to 1.5 equivalents, or 0.5 to 1 equivalent, relative to the copper complex compound of Formula 1. In addition, when [¹⁹F]fluoride ion is used as the fluoride ion in the reaction, the base may be added in an amount of 1 to 4 equivalents, or 1 to 3 equivalents, relative to the copper complex compound of Formula 1.

In the present invention, the reaction may be performed at a temperature of 50 to 200°C, without being limited thereto. Preferably, when [¹⁸F] fluoride ion is used as the fluoride ion in the reaction, the reaction may be performed at a temperature of 100 to 200°C, preferably 125 to 175°C. In addition, when [¹⁹F] fluoride ion is used as the fluoride ion in the reaction, the reaction may be performed at a temperature of 50 to 200°C, preferably 50 to 150 °C or 60 to 80°C.

In the present invention, the reaction may be performed for 5 to 120 minutes, without being limited thereto. Preferably, when [¹⁸F]fluoride ion is used as the fluoride ion in the reaction, the reaction may be performed for 10 to 30 minutes. In addition, when [¹⁹F]fluoride ion is used as the fluoride ion in the reaction, the reaction may be performed for 30 to 100 minutes.

A trifluoromethylation method according to an example of the present invention may comprise a step of reacting the ligand with the copper complex compound of Formula 1 and a [¹⁹F]fluoride ion source. In this case, the [¹⁹F]fluoride ion source may be added in an amount of 1 to 3 equivalents relative to the copper complex compound of Formula 1. In addition, the reaction may be performed at a reaction temperature of 50 to 150°C for 30 minutes to 100 minutes in the presence of a base in an amount of 1 to 4 equivalents relative to the copper complex compound of Formula 1, without being limited thereto.

A trifluoromethylation method according to an example of the present invention may comprise a step of reacting the above-described ligand with the copper complex compound of Formula 1 and a [¹⁸F]fluoride ion source. In this case, the reaction may be performed at a reaction temperature of 100 to 200°C for 10 minutes to 30 minutes in the presence of a base in an amount of 0.5 to 1.5 equivalents relative to the copper complex compound of Formula 1, without being limited thereto.

Non-limiting examples of ligands substituted with a trifluoromethyl group according to the method of the present invention include, but are not limited to, compounds having the following structures.

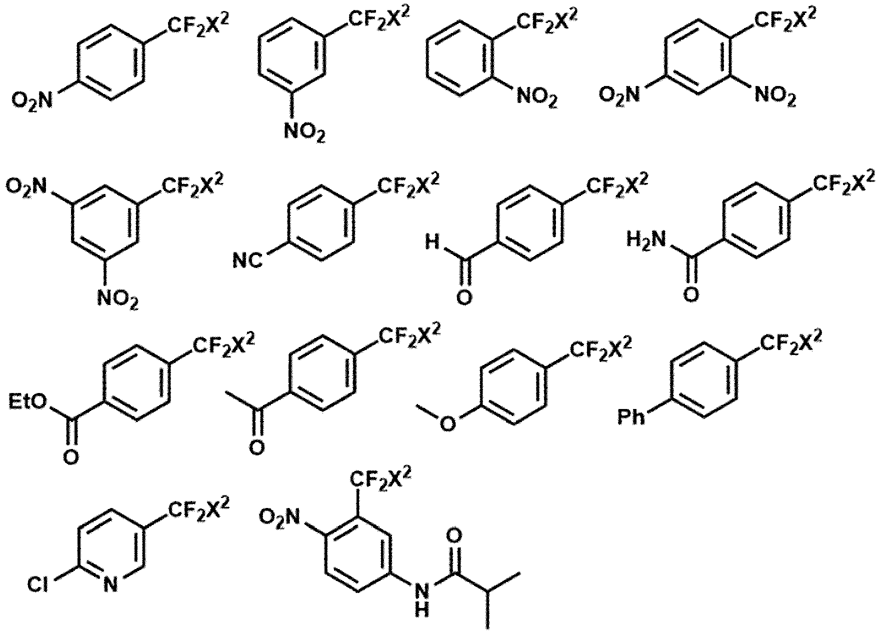

In the above formulas, X² is ¹⁸F or ¹⁹F.

### Trifluoroethylation Method

In the present invention, as the ligand to be substituted with the trifluoroethyl group, any ligand containing a primary amine group (-NH₂) or a secondary amine group (-NRH; where R may be any substituent) in its structure may be included without limitation as long as it may preferably be used for the purpose of diagnosing or treating a disease or for imaging.

In the present invention, non-limiting examples of the ligand containing the amine group include, but are not limited to, compounds such as N-methyl-3-phenylpropan-1-amine, N-methylbenzylamine, N,N-diethyl-amine, 1,2,3,4-tetrahydroisoquinoline, 1-methyl-4-piperazine, 4-morpholine, ethyl piperidine-2-carboxylate, piperidin-4-ol, N-(2-(6-methoxy)indolin-3-yl)ethyl)acetamide, indole, 6-bromoindoline, 4-methylindoline, N-methylbenzenamine, and the like.

The trifluoroethylation method of the present invention may comprise: a step of reacting the copper complex compound of Formula 1 with a fluoride ion source, thereby producing the trifluoroacetic acid represented by Formula 14; and a step of reacting the trifluoroacetic acid with the ligand.

In the trifluoroethylation method of the present invention, the step of producing the trifluoroacetic acid and the step of reacting the trifluoroacetic acid with the ligand may be performed as two separate steps, but may be performed continuously in one reactor.

In the present invention, any one hydrogen (H) of the amine group in the ligand may be substituted with a trifluoroethyl group derived from the trifluoroacetic acid of Formula 14 by a reaction represented by Reaction Scheme 2 below.

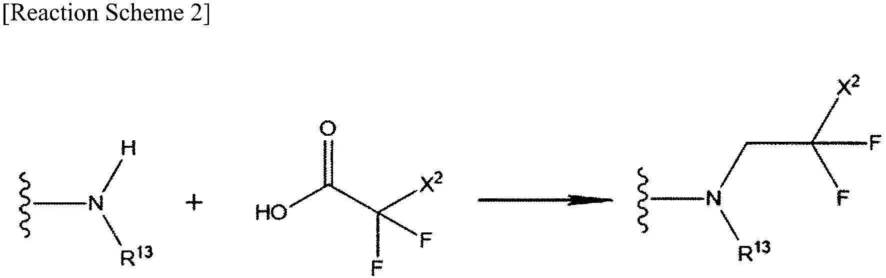

In Reaction Scheme 2 above, 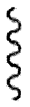
denotes a portion of the ligand to which the amine group is linked,
R₁₃ is hydrogen or any substituent, such as a C₁-C₆ alkyl group, a C₆-C₁₄ aryl group, or the like, which may vary depending on the structure of the ligand and is not particularly limited, and
Additionally, X² is ¹⁸F or ¹⁹F.

In the present invention, the reaction is preferably performed in the presence of a base so that the reaction efficiency may be increased. The base may be added in an amount of 1 to 4 equivalents, or 1 to 3 equivalents, relative to the copper complex compound of Formula 1, without being limited thereto.

In the present invention, the reaction of the copper complex compound represented by Formula 1 with the fluoride ion may be performed at a temperature of 50 to 100°C, preferably 60 to 80°C, more preferably 75°C, without being limited thereto.

In addition, in the present invention, the reaction of the copper complex compound represented by Formula 1 with the fluoride ion may be performed for 10 minutes to 6 hours, preferably 10 minutes to 1 hour, more preferably 10 minutes to 30 minutes, without being limited thereto.

In the present invention, the reaction of the trifluoroacetic acid of Formula 1 with the ligand containing the amine group may be performed in the presence of a reducing agent. Here, examples of the reducing agent that may be used include, but are not limited to, sodium borohydride (NaBH₄), sodium cyanoborohydride (NaCNBH₃), sodium acetate cyanoborohydride (NaBH₃OAc), sodium triacetoxyborohydride, lithium borohydride (LiBH₄), lithium aluminum hydride (LiAlH₄), (i-Bu₂AlH)₂, L-selectride, K-selectride, SnCl₂, phenylsilane, Et₃SiH, and mixtures thereof, with sodium cyanoborohydride (NaCNBH₃) being preferred. In the present invention, the reducing agent may be added in an amount of 1 to 5 equivalents, preferably 2 to 5 equivalents, relative to the ligand, without being limited thereto.

In the present invention, the reaction of the trifluoroacetic acid of Formula 1 with the ligand containing the amine group may be performed at 10 to 150°C, preferably 25 to 150°C, more preferably 100 to 150°C, most preferably 120°C, without being limited thereto.

In the present invention, the reaction of the trifluoroacetic acid of Formula 1 with the ligand containing the amine group may be performed for 5 to 120 minutes, preferably 15 to 60 minutes, more preferably 20 to 40 minutes, without being limited thereto.

Non-limiting examples of ligands substituted with a trifluoroethyl group according to the method of the present invention include, but are not limited to, compounds having the structures below:

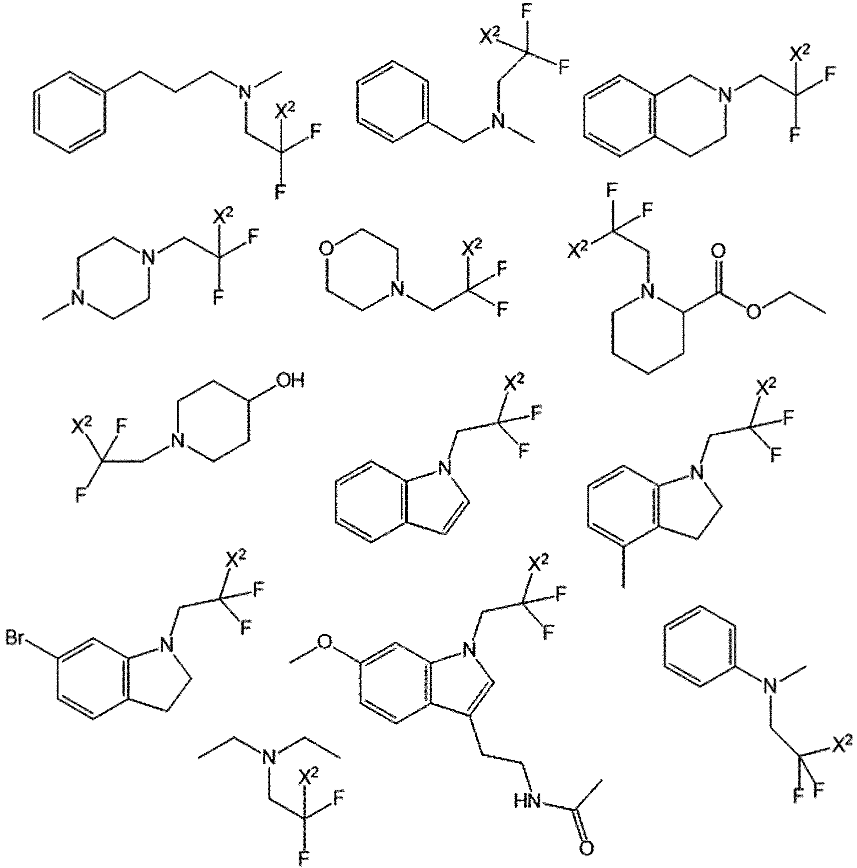

In the formulas above, X² is ¹⁸F or ¹⁹F.

According to the method of the present invention, a trifluoroalkyl group, particularly a trifluoromethyl group or a trifluoroethyl group, may be transferred to a target ligand, and when [¹⁸F] fluoride ion is used as the fluoride ion, a ligand having a fluorine-18-labeled trifluoroalkyl group introduced therein may be produced. The ligand having the -CF₂[¹⁸F] group, when administered *in vivo,* has excellent *in vivo* stability, and also has excellent lipophilicity and cell penetration ability.

According to another embodiment of the present invention, the present invention relates to a method of producing a radiopharmaceutical containing a ligand substituted with a [¹⁸F]fluoroalkyl group using the trifluoroalkylation method.

In the present invention, when [¹⁸F]fluoride ion is reacted as the fluoride ion, it is possible to produce a radiopharmaceutical substituted with fluorine-18-labeled trifluoromethyl (-CF₂[18^{F}]) or trifluoroethyl (-CH₂CF₂[¹⁸F]) by substituting the ligand with a [¹⁸F]trifluoromethyl group or a [¹⁸F]trifluoroethyl group.

According to another embodiment of the present invention, the present invention relates to a compound represented by any one of Formulas 16 to 30 below, or a pharmaceutically acceptable salt or solvate thereof, which is produced by the above method:

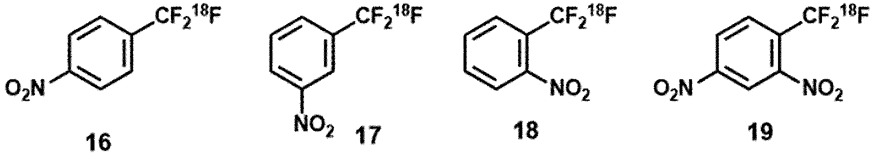

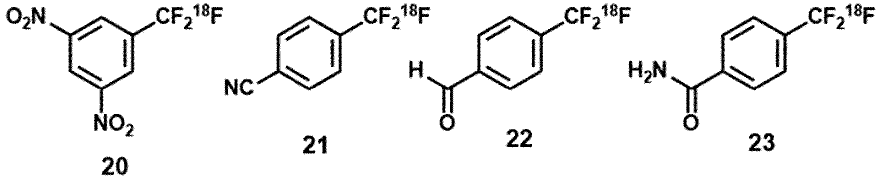

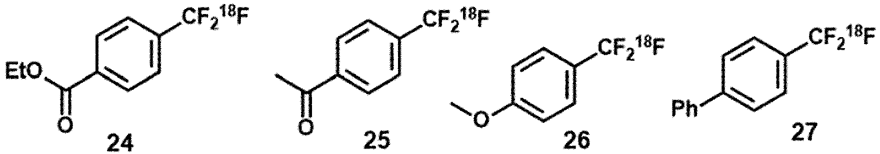

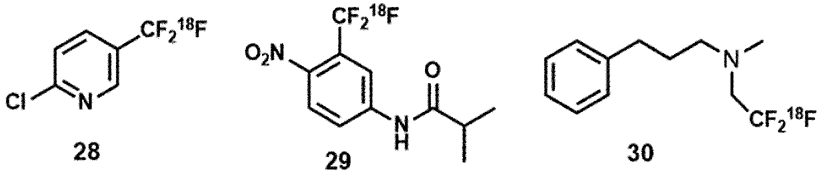

The present invention provides pharmaceutically acceptable salts of the compounds represented by Formulas 16 to 30. The pharmaceutically acceptable salts are salts which are usually considered by those skilled in the art to be suitable for medical applications, e.g., because they are not harmful to subjects which may be treated with the salts, or which give rise to side effects which are tolerable within the respective treatment. Usually, the pharmaceutically acceptable salts are salts which are considered as acceptable by the regulatory authorities, such as the US Food and Drug Administration (FDA), the European Medicines Agency (EMA), or the Japanese Ministry of Health, Labor and Welfare Pharmaceuticals and Medical Devices Agency (PMDA). However, the present invention in principle also encompasses salts of the compounds according to the present invention which are as such not pharmaceutically acceptable, e.g., as intermediates in the production of the compounds according to the present invention or physiologically functional derivatives thereof, or as intermediates in the production of pharmaceutically acceptable salts of the compounds according to the present invention or physiologically functional derivatives thereof. Said salts include water-insoluble salts and, particularly, water-soluble salts.

In each case, those skilled in the art can readily determine whether a certain compound according to the present invention or a physiologically functional derivative thereof can form a salt, i.e., whether the compound according to the present invention or a physiologically functional derivative thereof has a group which may carry a charge, such as, for example, an amino group, a carboxylic acid group, etc.

Exemplary salts of the compounds of the present invention are acid addition salts or salts with bases, particularly pharmaceutically acceptable inorganic and organic acid addition salts and salts with bases customarily used in pharmacy, which are either water insoluble or, particularly, water-soluble acid addition salts. Salts with bases may, depending on the substituents of the compounds of the present invention, also be suitable. Acid addition salts may, for example, be formed by mixing a solution of a compound of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid, or phosphoric acid. Likewise, pharmaceutically acceptable base addition salts may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, digluconate, dihydrochloride, dodecylsulfate, edetate, edisylate, ethanesulfonate, formate, fumarate, galactate, galacturonate, gluconate, glutamate, glycerophosphate, hemisulfate, heptanoate, hexanoate, hexylresorcinate, hydrobromide, hydrochloride, hydroiodide, 2-hydroxy-ethanesulfonate, hydroxynaphthoate, iodide, isobutyrate, isothionate, lactate, laurate, lauryl sulfate, malate, maleate, malonate, mandelate, methanesulfonate (mesylate), methylsulfate, 2-naphthalenesulfonate, nicotinate, nitrate, oleate, oxalate, palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, tosylate, undecanoate, valerate, and the like.

Salts, which are not pharmaceutically acceptable and which can be obtained, for example, as process products during the production of the compounds according to the present invention on an industrial scale, are also encompassed by the present invention and, if desired, may be converted into pharmaceutically acceptable salts by processes known to those skilled in the art.

Meanwhile, the compounds according to the present invention may have an asymmetric carbon center, and thus may exist as R or S isomers or racemic compounds, and all of these optical isomers and mixtures may be included in the scope of the present invention.

In addition, the compounds of the present invention as well as salts thereof may contain various amounts of solvent when isolated, for example, in crystalline form. Therefore, solvates and in particular hydrates of the compounds of the present invention, and also solvates and in particular hydrates of the salts of the compounds of the present invention may be included within the scope of the present invention. More particularly, the present invention may include hydrates of the compounds, salts, and/or physiologically functional derivatives according to the present invention, which comprise one, two or one half water molecule, with respect to their stoichiometry.

According to another embodiment of the present invention, the present invention relates to a pharmaceutical composition for preventing, alleviating or treating a disease, comprising a compound represented by Formula 29 below or a pharmaceutically acceptable salt or solvate thereof as an active ingredient:

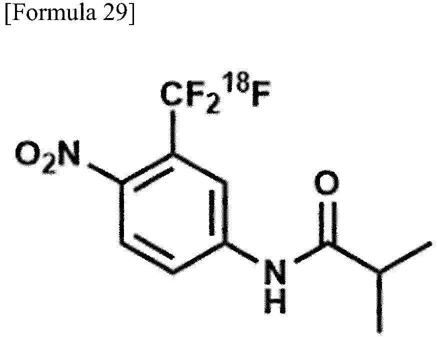

According to another embodiment of the present invention, the present invention relates to a method for preventing, alleviating or treating a disease, comprising administering to a subject in need of administration a pharmaceutically effective amount of the compound represented by Formula 29, or a pharmaceutically acceptable salt or solvate thereof.

In the present invention, the disease may be cancer. The cancer may be melanoma, fallopian tube cancer, brain cancer, small intestine cancer, esophageal cancer, lymphoma, gallbladder cancer, blood cancer, thyroid cancer, endocrine cancer, oral cancer, liver cancer, biliary tract cancer, colon cancer, rectal cancer, cervical cancer, ovarian cancer, kidney cancer, stomach cancer, duodenal cancer, prostate cancer, breast cancer, brain tumor, lung cancer, undifferentiated thyroid cancer, uterine cancer, colon cancer, bladder cancer, ureteral cancer, pancreatic cancer, bone/soft tissue sarcoma, skin cancer, non-Hodgkin's lymphoma, Hodgkin's lymphoma, multiple myeloma, leukemia, myelodysplastic syndrome, acute lymphoblastic leukemia, acute myelogenous leukemia, chronic lymphocytic leukemia, chronic myelogenous leukemia, or solitary myeloma, and is preferably prostate cancer, without being limited thereto.

In the present invention, the subject is a subject having or suspected of having the above-listed diseases. The subject suspected of having the disease means all animals including humans, monkeys, cows, horses, sheep, pigs, chickens, turkeys, quails, cats, dogs, mice, rats, rabbits or guinea pigs that have or are likely to develop the disease, but includes, without limitation, subjects that can be treated with the active ingredient provided by the present invention.

In the present invention, the "pharmaceutical composition" may be characterized in that it is in the form of capsules, tablets, granules, injections, ointments, powders, or beverages, and may also be characterized in that the pharmaceutical composition is for administration to animals, specifically humans.

For use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition according to the present invention may contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be prepared in the form of tablets, troches, capsules, elixirs, suspensions, syrups, wafers, or the like. For injection, the pharmaceutical composition may be prepared in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, or mineral oil. In addition, the pharmaceutical composition of the present invention may further contain a filler, an anticoagulant, a lubricant, a wetting agent, a fragrance, an emulsifier, a preservative, or the like.

In the present invention, the pharmaceutical composition may further comprise a radical scavenger in addition to the compound of the present invention or a pharmaceutically acceptable salt thereof. The radical scavenger may be used to prevent radiolysis. Radiolysis is the process in which the ionization of oxygen or water molecules induced by the radionuclide leads to the formation of other reactive species, such as superoxide, hydrogen peroxide, hydrogen radicals, ozone and hydroxyl radicals. These reactive species can further cause damage to DNA and other cellular structures. In some embodiments, the radical scavenger is an antioxidant selected from carnosic acid, green tea extract, apigenin, diosmine, rosmarinic acid, lipoic acid, beta carotene, L-ascorbic acid (vitamin C), N-acetyl cysteine (NAC), δ-tocopherol, rutin, amifostine, resveratrol, gentisic acid, and gallic acid. In some embodiments, the radical scavenger may be an antioxidant selected from gallic acid, L-ascorbic acid and N-acetyl cysteine (NAC), without being limited thereto.

The routes of administration of the pharmaceutical composition according to the present invention include, but are not limited to, oral, intravenous, intramuscular, intraarterial, intramedullary, intradural, intracardiac, transdermal, subcutaneous, intraperitoneal, intranasal, enteral, topical, sublingual, or rectal administration. Oral or parenteral administration is preferred. The "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be formulated as suppositories for intrarectal administration.

The pharmaceutical composition may vary depending on a variety of factors, including the activity of a specific compound used, the patient's age, body weight, general health status, sex and diet, the time of administration, the route of administration, the rate of excretion, drug combination, and the severity of a specific disease to be prevented or treated. Although the dosage of the pharmaceutical composition may vary depending on the patient's condition and body weight, the severity of disease, the drug form, and the route and duration of administration, it may be appropriately selected by those skilled in the art, and the pharmaceutical composition may be administered at a dose of 0.0001 to 50 mg/kg/day or 0.001 to 50 mg/kg/day. The administration may be done once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

The pharmaceutical composition of the present invention may be used alone or in combination with surgery, radiotherapy, hormone therapy, chemotherapy, and methods that use biological response modifiers.

According to another embodiment of the present invention, the present invention relates to an imaging composition or a composition for diagnosing a disease, comprising, as an active ingredient, a compound represented by any one of Formulae 16 to 30 above, or a pharmaceutically acceptable salt or solvate thereof.

According to another embodiment of the present invention, the present invention relates to an imaging method or a method for diagnosing a disease, comprising a step of administering to a subject in need of administration a pharmaceutically effective amount of a compound represented by any one of Formulae 16 to 30 above, or a pharmaceutically acceptable salt or solvate thereof.

In the present invention, the subject is a subject having or suspected of having the disease of interest and requiring tissue imaging, and means all animals including humans, monkeys, cows, horses, sheep, pigs, chickens, turkeys, quails, cats, dogs, mice, rats, rabbits or guinea pigs that have or are likely to develop the disease, but includes, without limitations, subjects that can be treated with the active ingredient by the present invention.

In the present invention, when a pharmaceutical composition containing the compound according to the present invention is administered to a subject of interest, the compound binds to a lesion tissue, such as a tumor tissue or cancer tissue, thereby enabling imaging. In the present invention, the technique for imaging may be, but is not limited to, positron emission tomography (PET), or a combination of positron emission tomography and computed tomography (PET-CT).

Therefore, when the compound of the present invention is used, it is possible to diagnose whether or not a subject has or is likely to develop a disease such as cancer or tumor.

### Advantageous Effects

When the copper complex compound provided in the present invention is used, a target ligand may be substituted with a trifluoroalkyl group in high yield. In addition, when [¹⁸F] fluoride ion is used as the fluoride ion in the reaction, the ligand may be substituted with a -CF₂[¹⁸F] group. A radiopharmaceutical substituted with a -CF₂[¹⁸F] group as described above has the advantages of having excellent *in vivo* drug stability, lipophilicity, and cell penetration ability.

### Brief Description of Drawings

FIG. 1 shows the results of analyzing the amount of trifluoroacetic acid produced in a reaction mixture depending on the time of reaction of a copper complex compound with [¹⁹F]fluoride ion by ¹⁹F-NMR in Experimental Example 2.
FIG. 2 shows the results of analyzing a reaction mixture of a copper complex compound and [¹⁸F] fluoride ion by HPLC in Experimental Example 4.
FIG. 3 shows the results of analyzing a waste, obtained after passing a reaction mixture of a copper complex compound and [¹⁸F] fluoride ion through a Sep-pak cartridge, by HPLC in Experimental Example 5.
FIG. 4 shows the results of analyzing a waste, obtained after passing a reaction mixture of a copper complex compound and [¹⁸F] fluoride ion through a Sep-pak cartridge, followed by passage through a WAX cartridge, by HPLC in Experimental Example 5.
FIG. 5 shows the results of analyzing a product, obtained after reacting a ligand containing an amine group with ¹⁸F-TFA, by HPLC in Experimental Example 6.
FIG. 6 shows the results of analyzing a product, obtained after reacting a ligand containing a halogen-substituted aryl group with a copper complex compound and [¹⁸F] fluoride ion, by HPLC in Experimental Example 7.

### Best Mode

According to one embodiment of the present invention, the present invention relates to a building block composition for trifluoroalkylation comprising a copper complex compound represented by Formula 1 below.

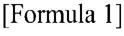

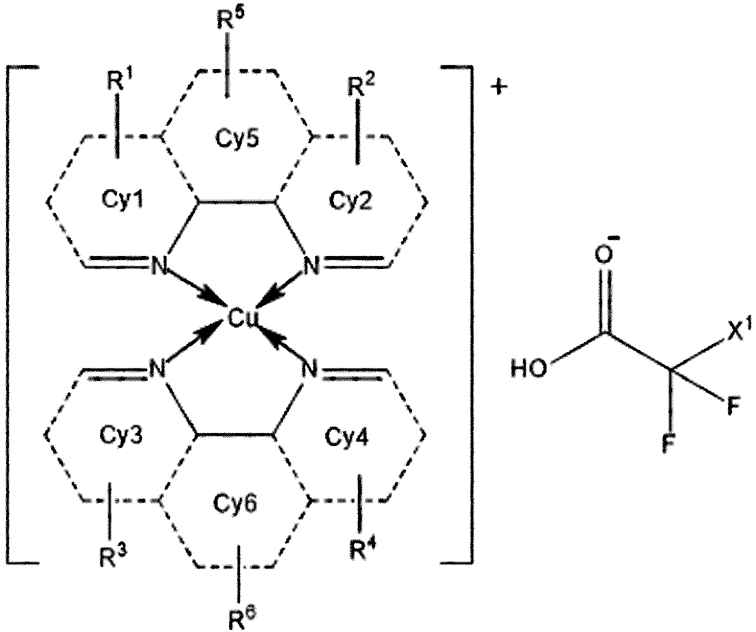

In Formula 1 above,
Cy1 to Cy4 are each independently a 4- to 8-membered heteroaryl or heterocycloalkenyl ring,
Cy5 and Cy6 are each independently a 0- to 8-membered cycloalkyl, cycloalkenyl, aryl, heteroaryl, heterocycloalkyl or heterocycloalkenyl ring (wherein 0-membered means there is no ring),
R¹ to R⁶ are each independently hydrogen, a halogen, nitro, a C₁-C₆ alkyl group, or a C₆-C₁₄ aryl group, and are mono-substituents or the same or different multi-substituents, and
X¹ may be a halogen other than a fluoro group.

In the present invention, the copper complex compound represented by Formula 1 may be a compound represented by Formula 6 or 7 below, without being limited thereto.

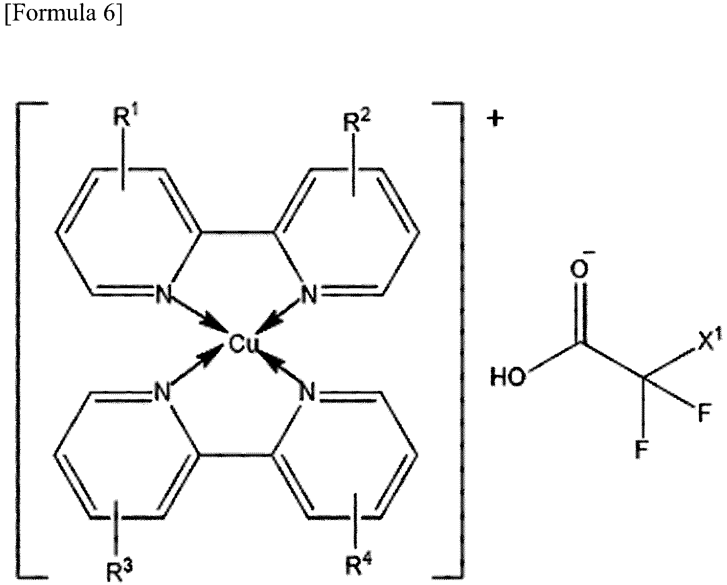

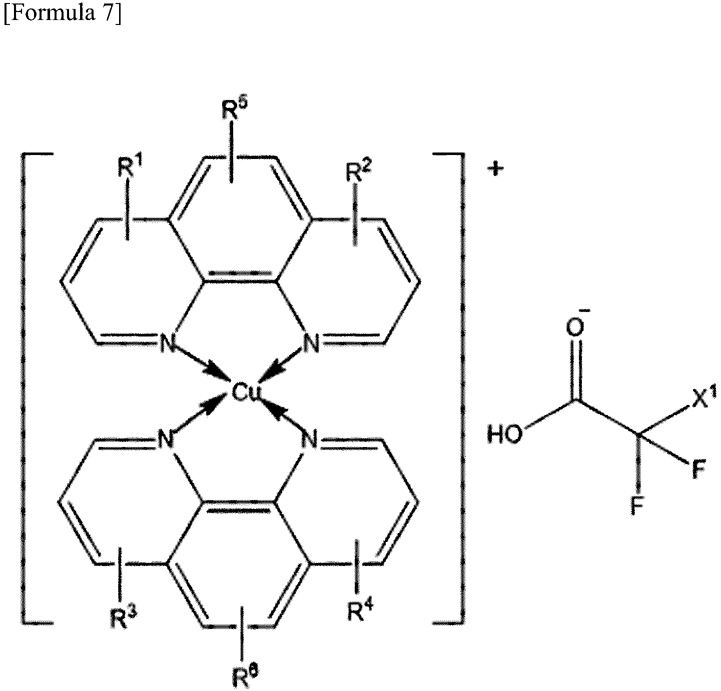

In Formulas 6 and 7 above, R¹ to R⁶ and X¹ are as defined above.

In the present invention, the copper complex compound represented by Formula 1 may be a compound represented by any one of Formulas 11 to 13 below, without being limited thereto.

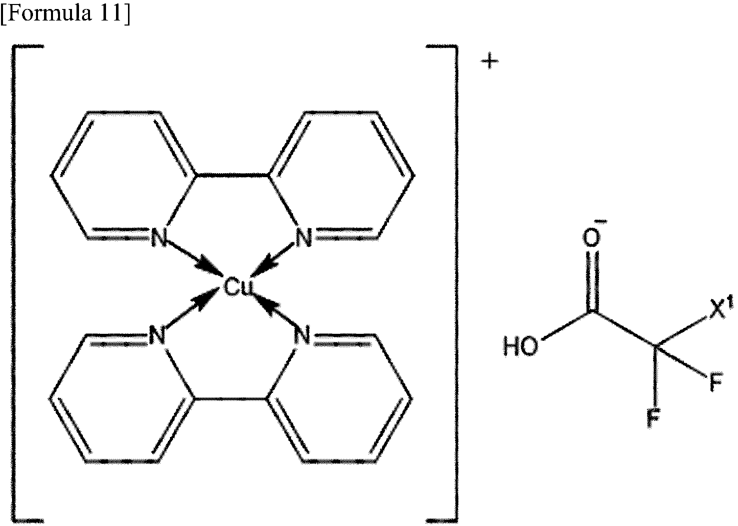

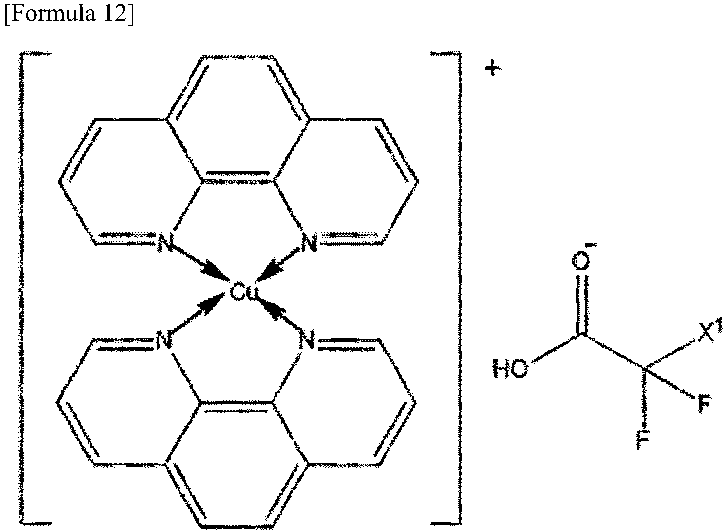

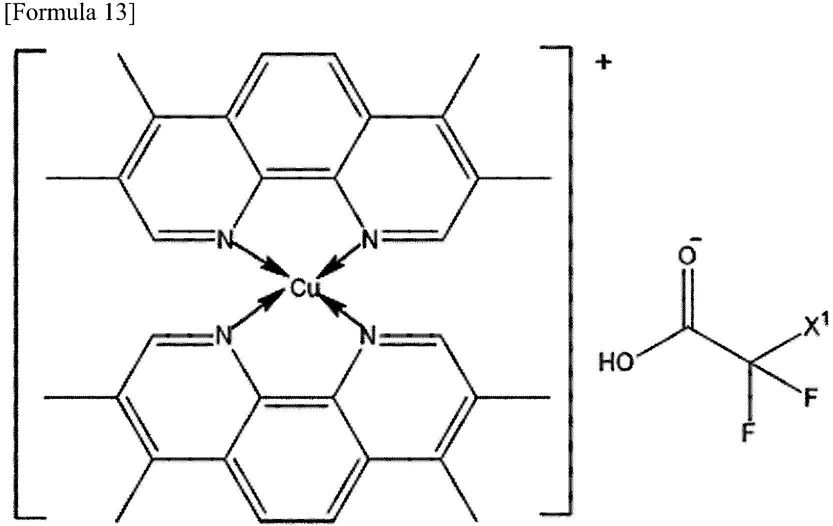

In Formulas 11 to 13 above, X¹ is as defined above.

According to another embodiment of the present invention, the present invention relates to a method of producing a trifluoroacetic acid represented by Formula 14 below by reacting a compound represented by Formula 2 below or the copper complex compound represented by Formula 1 with [¹⁸F]fluoride ion or [¹⁹F]fluoride ion:

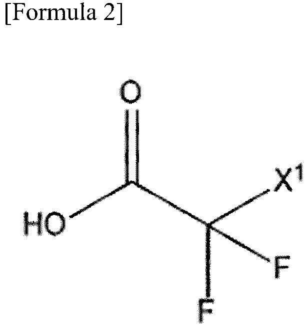

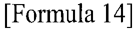

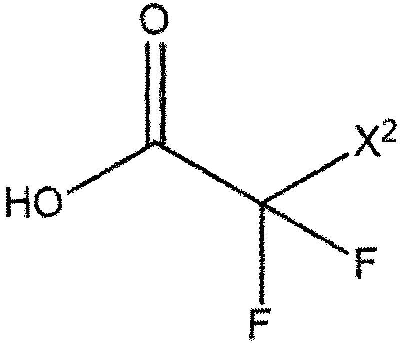

In Formulas 2 and 14 above, X¹ is as defined above, and X² is ¹⁸F or ¹⁹F.

In the present invention, the trifluoroacetic acid of Formula 14 may be produced by reacting the compound of Formula 2 or the copper complex compound represented by Formula 1 with fluoride ion.

In the present invention, the copper complex compound of Formula 1 may be produced by reacting the compound of Formula 2 with the copper complex represented by Formula 3 as described above.

In the present invention, the [¹⁸F]fluoride ion or [¹⁹F]fluoride ion may be added in an amount of 0.2 to 4 equivalents, preferably 0.2 to 2 equivalents, more preferably 0.2 to 1.5 equivalents, most preferably 1 equivalent, relative to the copper complex compound represented by Formula 1, without being limited thereto.

In the present invention, the reaction of the compound represented by Formula 2 or the copper complex compound represented by Formula 1 with [¹⁸F]fluoride ion or [¹⁹F]fluoride ion is preferably performed in the presence of a base so that the reaction efficiency may be increased. Here, as the base, alkoxides, such as sodium tert-butoxide; an alkali metal amide, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 2 equivalents, preferably 0.75 to 1.5 equivalents, most preferably 1 equivalent, relative to the compound represented by Formula 2 or 4, without being limited thereto.

In the present invention, the reaction of the compound represented by Formula 2 or the copper complex compound of Formula 1 with the fluoride ion may be performed in a solvent. Here, as the solvent, any one or more selected from the group consisting of CH₃CN, tetrahydrofuran (THF), CH₂Cl₂, N,N-dimethylformamide (DMF), dimethyl sulfoxide (DMSO), and mixtures thereof may be used, without being limited thereto.

In addition, in the present invention, the reaction of the compound represented by Formula 2 or the copper complex compound represented by Formula 1 with the fluoride ion may be performed at a temperature of 50 to 100°C, preferably 60 to 80°C, more preferably 75°C, without being limited thereto.

In addition, in the present invention, the reaction of the compound represented by Formula 2 or the copper complex compound represented by Formula 1 with the fluoride ion may be performed for 10 minutes to 6 hours, preferably 10 minutes to 1 hour, more preferably 10 minutes to 30 minutes, without being limited thereto.

In the present invention, the [¹⁸F]fluoride ion may be obtained through the 18O(p,n)¹⁸F reaction from a cyclotron, without being limited thereto. In the present invention, if necessary, a step of obtaining a [¹⁸F]F⁻/H₂¹⁸O solution by separating the [¹⁸F]fluoride obtained through the above reaction using an acetonitrile reaction solution containing K_{2.2.2} and K₂CO₃ dissolved therein, and a step of obtaining K_{2.2.2}/K[¹⁸F]F by heating the [¹⁸F]F⁻/H₂¹⁸O solution may be additionally performed, and the obtained K_{2.2.2}/K[¹⁸F]F may be used.

In addition, in the present invention, the [¹⁸F] fluoride ion may be used after moisture is removed therefrom by adding [¹⁸F] fluoride to a reaction vessel together with a phase transfer catalyst and a base additive under a solvent, and then blowing nitrogen at a temperature of 80 to 100°C. Here, the solvent may be methyl alcohol, acetonitrile, or a mixture thereof. As the phase transfer catalyst, Kryptofix_{2.2.2} (K_{2.2.2}) or 18-crown-6 may be used, and as the basic additive, one selected from the group consisting of CsHCO₃, Cs₂CO₃, KHCO₃, K₂CO₃ and tetrabutylammonium salt may be used, without being limited thereto.

The production method of the present invention may, if necessary, further comprise a step of separating and purifying trifluoroacetic acid from the reaction mixture obtained after the reaction. In the present invention, the separation and purification method is not particularly limited, but may be performed by passing the reaction mixture through a cartridge containing an alumina adsorbent (sorbent substrate), and for example, may be performed using a Sep-pak^{®} alumina N cartridge. Preferably, in terms of the separation and purification efficiency of the trifluoroacetic acid, the reaction mixture may be passed through the cartridge filled with 250 to 2,000 mg of the alumina adsorbent, and more preferably, may be passed through the cartridge filled with 1,000 to 2,000 mg of the alumina adsorbent. As a specific example, an alumina N light cartridge filled with 280 mg of the alumina adsorbent may be used, or an alumina N plus cartridge filled with 1,710 mg of the alumina adsorbent may be used, and more preferably, an alumina N plus cartridge may be used, without being limited thereto.

In the present invention, the [¹⁹F] fluoride ion may be derived from a metal fluoride compound, without being limited thereto, and any fluoride source capable of supplying [¹⁹F] fluoride ion may be included without limitation. Here, examples of the metal fluoride compound that may be used include, but are not limited to, LiF, NaF, KF, CsF, RbF, TiF, AgF, AgF₂, BaF₂, CaF₂, CuF₂, CdF₂, FeF₂, HgF₂, Hg₂F₂, MnF₂, MgF₂, NiF₂, PbF₂, SnF₂, SrF₂, XeF₂, ZnF₂, AlF₂, BF₂, BiF₂, CeF₂, CrF₃, DyF₃, EuF₃, GaF₃, GdF₃, FeF₃, HoF₃, InF₃, LaF₃, LuF₃, MnF₃, NdF₃, VOF₃, PrF₃, SbF₃, ScF₃, SmF₃, TbF₃, TiF₃, TmF₃, YF₃, YbF₃, TIF₃, CeF₄, GeF₄, HfF₄, SiF₄, SnF₄, TiF₄, VF₄, ZrF₄, NbF₅, SbF₅, TaF₅, BiF₅, MoF₆, ReF₆, SF₆ and WF₆.

The production method of the present invention may, if necessary, further comprise a step of formulating the reaction mixture obtained after the reaction. In the present invention, the formulation method is not particularly limited, but may be performed by passing the reaction mixture through a cartridge containing a copolymer sorbent, and for example, may be performed using an Oasis WAX Plus short cartridge, without being limited thereto.

When the production method of the present invention is used, it is possible to produce trifluoroacetic acid in high yield. In the present invention, trifluoroacetic acid labeled with fluorine-18 may be produced by reacting the compound represented by Formula 2 or the copper complex compound of Formula 1 with [¹⁸F] fluoride ion, and the fluorine-18-labeled trifluoroacetic acid produced as described above may be used for the production of additional radiopharmaceuticals.

According to another embodiment of the present invention, the present invention relates to a method of producing a trifluoroacetic acid represented by Formula 14 below by reacting the compound represented by Formula 2 or the copper complex compound represented by Formula 1 with [¹⁸F]fluoride ion or [¹⁹F]fluoride ion:

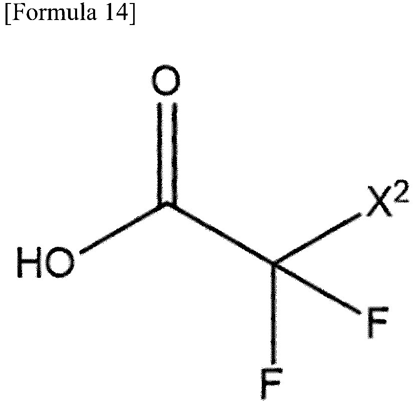

In Formula 14 above, X² is ¹⁸F or ¹⁹F.

In the present invention, the trifluoroacetic acid of Formula 14 may be produced by reacting each of the compound of Formula 2 and the copper complex compound represented by Formula 1 with fluoride ion.

In the present invention, the copper complex compound of Formula 1 may be produced by reacting the compound of Formula 2 with the copper complex represented by Formula 3 as described above.

In the present invention, the [¹⁸F]fluoride ion or [¹⁹F]fluoride ion may be added in an amount of 0.2 to 4 equivalents, preferably 0.2 to 2 equivalents, more preferably 0.2 to 1.5 equivalents, most preferably 1 equivalent, relative to the copper complex compound represented by Formula 1, without being limited thereto.

In the present invention, the reaction of the compound represented by Formula 2 or the copper complex compound represented by Formula 1 with [¹⁸F]fluoride ion or [¹⁹F]fluoride ion is preferably performed in the presence of a base so that the reaction efficiency may be increased. Here, as the base, alkoxides, such as sodium tert-butoxide; an alkali metal amide, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 2 equivalents, preferably 0.75 to 1.5 equivalents, most preferably 1 equivalent, relative to the compound represented by Formula 2 or 4, without being limited thereto.

In addition, in the present invention, the reaction of the compound represented by Formula 2 or the copper complex compound represented by Formula 1 with the fluoride ion may be performed at a temperature of 50 to 100°C, preferably 60 to 80°C, more preferably 75°C, without being limited thereto.

In addition, in the present invention, the reaction of the compound represented by Formula 2 or the copper complex compound represented by Formula 1 with the fluoride ion may be performed for 10 minutes to 6 hours, preferably 10 minutes to 1 hour, more preferably 10 minutes to 30 minutes, without being limited thereto.

When the production method of the present invention is used, it is possible to produce trifluoroacetic acid in high yield. In the present invention, trifluoroacetic acid labeled with fluorine-18 may be produced by reacting the compound represented by Formula 2 or the copper complex compound of Formula 1 with [¹⁸F] fluoride ion, and the fluorine-18-labeled trifluoroacetic acid produced as described above may be used for the production of additional radiopharmaceuticals.

According to another embodiment of the present invention, the present invention relates to a method of trifluoroalkylating a ligand using the copper complex compound represented by Formula 1.

The trifluoroalkylation method of the present invention may comprise a step of reacting a ligand with the copper complex compound of Formula 1 in the presence of a fluoride ion, thereby trifluoroalkylating the ligand.

In the present invention, the fluoride ion may be [¹⁸F]fluoride ion or [¹⁹F]fluoride ion depending on the purpose. When [¹⁸F]fluoride ion is used as the fluoride ion, the ligand may be labeled with the radioactive isotope fluorine-18.

In the present invention, the fluoride ion may be added in an amount of 0.2 to 4 equivalents, preferably 0.2 to 3 equivalents, more preferably 1 to 3 equivalents, relative to the copper complex compound represented by Formula 1, without being limited thereto.

In the present invention, the reaction is preferably performed in the presence of a base so that the reaction efficiency may be increased. Here, as the base, alkoxides, such as sodium tert-butoxide; alkali metal amides, such as sodium amide, lithium diisopropylamide, and alkali metal bis(trialkylsilyl)amides, such as lithium bis(trimethylsilyl)amide (LiHMDS) or sodium bis(trimethylsilyl)amide (NaHMDS); tertiary amines (e.g., triethylamine, trimethylamine, 4-(dimethylamino)pyridine (DMAP), 1,5-diazabicyclo[4.3.0]non-5-ene (DBN), or 1,5-diazabicyclo[5.4.0]undec-5-ene (DBU); alkali or alkaline earth carbonates, bicarbonates or hydroxides (e.g., sodium, magnesium, calcium, barium or potassium carbonate, phosphate, hydroxide and bicarbonate), etc. may be used, and preferably, NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or mixtures thereof may be used, without being limited thereto. In the present invention, the base may be added in an amount of 0.5 to 4 equivalents, preferably 0.5 to 3 equivalents, relative to the copper complex compound of Formula 1, without being limited thereto.

In the present invention, the reaction of the copper complex compound of Formula 1 with the fluoride ion may be performed in a solvent. Here, as the solvent, any one or more selected from the group consisting of CH₃CN, tetrahydrofuran (THF), CH₂Cl₂, N,N-dimethylformamide (DMF), dimethyl sulfoxide (DMSO), and mixtures thereof may be used, without being limited thereto.

In the present invention, even if the same copper complex compound of Formula 1 is used, the ligand may be substituted with a trifluoromethyl group or a trifluoroethyl group depending on the ligand and the reaction conditions.

### Trifluoromethylation Method

In the present invention, as the ligand to be substituted with the trifluoromethyl group, any ligand containing an aryl (e.g., C₆-C₁₄ aryl) or heteroaryl (e.g., heteroaryl having 5 to 14 nuclear atoms) structure substituted with at least one of a halogen, a boronic acid group (-B(OH)₂), or a boronic acid pinacol ester group (-Bpin) may be included without limitation as long as it may preferably be used for the purpose of diagnosing or treating a disease or for imaging.

In the present invention, the ligand containing the halogen-substituted aryl or heteroaryl structure may contain a phenyl structure substituted with a fluoro group, a chloro group, a bromo group, an iodo group, a boronic acid group (-B(OH)₂), or a boronic acid pinacol ester group (-Bpin), and may be, for example, a compound represented by Formula 15 below, without being limited thereto.

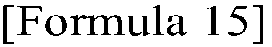

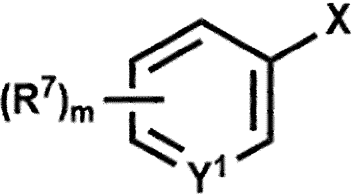

In Formula 15 above,
X is a halogen, a boronic acid group or a boronic acid pinacol ester group, preferably a halogen,
Y¹ is N or C(R⁸),
m is an integer ranging from 0 to 4,
R⁷ is a halogen, nitro, a cyano group, a C₁-C₆ alkyl group, a C₆-C₁₄ aryl group, a C₁-C₆ alkoxy group, -C(=O)-R⁹, or -NR¹⁰C(=O)-R¹¹, and when there are a plurality of R⁷s, they are the same as or different from each other,
R⁸ is hydrogen or a C₁-C₆ alkyl group,
R⁹ is hydrogen, an amine group (-NH₂), a C₁-C₆ alkyl group, or a C₁-C₆ alkoxy group, and
R¹⁰ and R¹¹ are each independently hydrogen or a C₁-C₆ alkyl group.

In the present invention, non-limiting examples of the ligand including the halogen-substituted aryl or heteroaryl structure include, but are not limited to, compounds having the following structures:

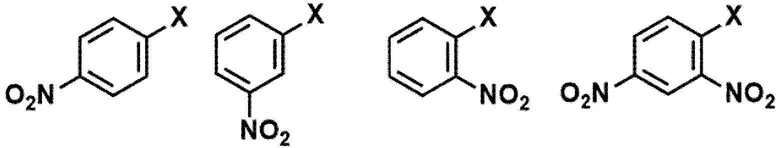

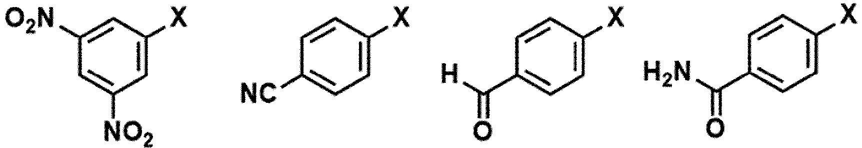

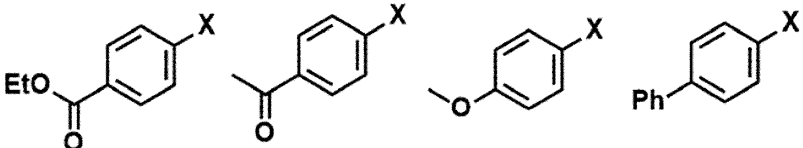

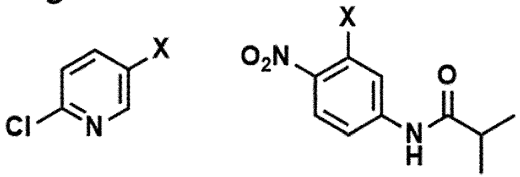

In the above formulas, X is a halogen, a boronic acid group, or a boronic acid pinacol ester group.

The trifluoromethylation method of the present invention may comprise a step of reacting the above-described ligand with the copper complex compound of Formula 1 and a fluoride ion source.

In the present invention, the halogen, boronic acid group or boronic acid pinacol ester group in the ligand may be substituted with a trifluoromethyl group derived from the compound of Formula 1 by a reaction represented by Reaction Scheme 1 below.

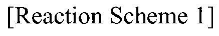

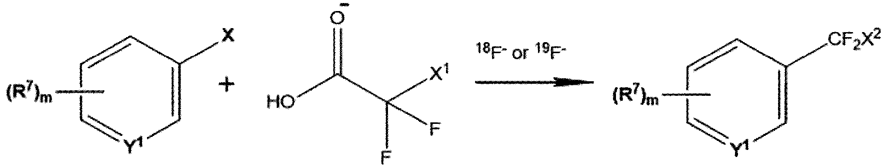

In Reaction Scheme 1 above, each group is as defined above.

In the present invention, the fluoride ion may be added in an amount of 0.2 to 4 equivalents, preferably 1 to 3 equivalents, relative to the copper complex compound represented by Formula 1, without being limited thereto.

In the present invention, the reaction is preferably performed in the presence of a base so that the reaction efficiency may be increased. The base may be added in an amount of 0.5 to 4 equivalents, or 0.5 to 3 equivalents, relative to the copper complex compound of Formula 1. Preferably, when [¹⁸F]fluoride ion is used as the fluoride ion in the reaction, the base may be added in an amount of 0.5 to 1.5 equivalents, or 0.5 to 1 equivalent, relative to the copper complex compound of Formula 1. In addition, when [¹⁹F]fluoride ion is used as the fluoride ion in the reaction, the base may be added in an amount of 1 to 4 equivalents, or 1 to 3 equivalents, relative to the copper complex compound of Formula 1.

In the present invention, the reaction may be performed at a temperature of 50 to 200°C, without being limited thereto. Preferably, when [¹⁸F] fluoride ion is used as the fluoride ion in the reaction, the reaction may be performed at a temperature of 100 to 200°C, preferably 125 to 175°C. In addition, when [¹⁹F] fluoride ion is used as the fluoride ion in the reaction, the reaction may be performed at a temperature of 50 to 200°C, preferably 50 to 150 °C or 60 to 80°C.

In the present invention, the reaction may be performed for 5 to 120 minutes, without being limited thereto. Preferably, when [¹⁸F]fluoride ion is used as the fluoride ion in the reaction, the reaction may be performed for 10 to 30 minutes. In addition, when [¹⁹F]fluoride ion is used as the fluoride ion in the reaction, the reaction may be performed for 30 to 100 minutes.

A trifluoromethylation method according to an example of the present invention may comprise a step of reacting the ligand with the copper complex compound of Formula 1 and a [¹⁹F]fluoride ion source. In this case, the [¹⁹F]fluoride ion source may be added in an amount of 1 to 3 equivalents relative to the copper complex compound of Formula 1. In addition, the reaction may be performed at a reaction temperature of 50 to 150°C for 30 minutes to 100 minutes in the presence of a base in an amount of 1 to 4 equivalents relative to the copper complex compound of Formula 1, without being limited thereto.

A trifluoromethylation method according to an example of the present invention may comprise a step of reacting the above-described ligand with the copper complex compound of Formula 1 and a [¹⁸F]fluoride ion source. In this case, the reaction may be performed at a reaction temperature of 100 to 200 °C for 10 minutes to 30 minutes in the presence of a base in an amount of 0.5 to 1.5 equivalents relative to the copper complex compound of Formula 1, without being limited thereto.

Non-limiting examples of ligands substituted with a trifluoromethyl group according to the method of the present invention include, but are not limited to, compounds having the following structures.

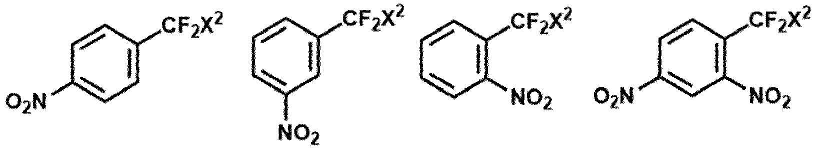

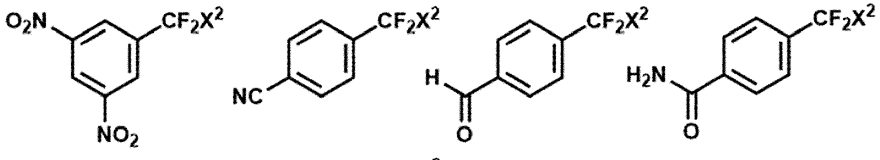

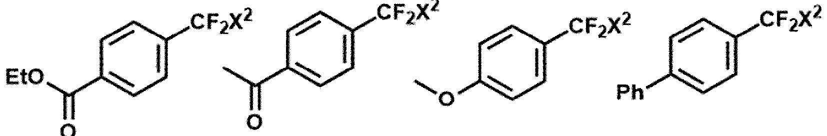

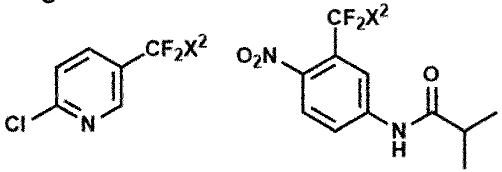

In the above formulas, X² is ¹⁸F or ¹⁹F.

### Trifluoroethylation Method

In the present invention, as the ligand to be substituted with the trifluoroethyl group, any ligand containing a primary amine group (-NH₂) or a secondary amine group (-NRH; where R may be any substituent) in its structure may be included without limitation as long as it may preferably be used for the purpose of diagnosing or treating a disease or for imaging.

In the present invention, non-limiting examples of the ligand containing the amine group include, but are not limited to, compounds such as N-methyl-3-phenylpropan-1-amine, N-methylbenzylamine, N,N-diethyl-amine, 1,2,3,4-tetrahydroisoquinoline, 1-methyl-4-piperazine, 4-morpholine, ethyl piperidine-2-carboxylate, piperidin-4-ol, N-(2-(6-methoxy)indolin-3-yl)ethyl)acetamide, indole, 6-bromoindoline, 4-methylindoline, N-methylbenzenamine, and the like.

The trifluoroethylation method of the present invention may comprise: a step of producing the trifluoroacetic acid represented by Formula 14 by reacting the copper complex compound of Formula 1 with a fluoride ion source; and a step of reacting the trifluoroacetic acid with the ligand.

In the trifluoroethylation method of the present invention, the step of producing the trifluoroacetic acid and the step of reacting the trifluoroacetic acid with the ligand may be performed as two separate steps, but may be performed continuously in one reactor.

In the present invention, any one hydrogen (H) of the amine group in the ligand may be substituted with a trifluoroethyl group derived from the trifluoroacetic acid of Formula 14 by a reaction represented by Reaction Scheme 2 below.

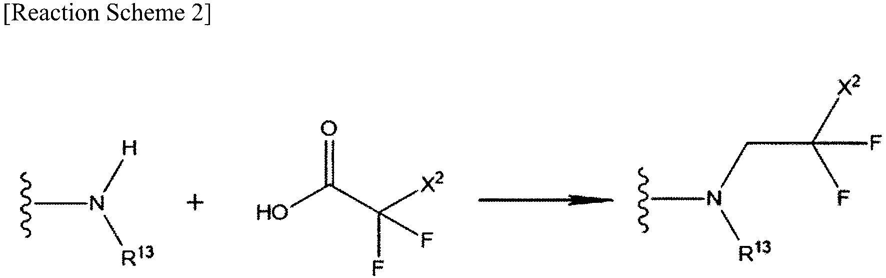

In Reaction Scheme 2 above, 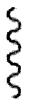
denotes a portion of the ligand to which the amine group is linked,
R₁₃ is hydrogen or any substituent, such as a C₁-C₆ alkyl group, a C₆-C₁₄ aryl group, or the like, which may vary depending on the structure of the ligand and is not particularly limited, and
X² is ¹⁸F or ¹⁹F.

In the present invention, the reaction is preferably performed in the presence of a base so that the reaction efficiency may be increased. The base may be added in an amount of 1 to 4 equivalents, or 1 to 3 equivalents, relative to the copper complex compound of Formula 1, without being limited thereto.

In the present invention, the reaction of the copper complex compound represented by Formula 1 with the fluoride ion may be performed at a temperature of 50 to 100°C, preferably 60 to 80°C, more preferably 75°C, without being limited thereto.

In addition, in the present invention, the reaction of the copper complex compound represented by Formula 1 with the fluoride ion may be performed for 10 minutes to 6 hours, preferably 10 minutes to 1 hour, more preferably 10 minutes to 30 minutes, without being limited thereto.

In the present invention, the reaction of the trifluoroacetic acid of Formula 1 with the ligand containing the amine group may be performed in the presence of a reducing agent. Here, examples of the reducing agent that may be used include, but are not limited to, sodium borohydride (NaBH₄), sodium cyanoborohydride (NaCNBH₃), sodium acetate cyanoborohydride (NaBH₃OAc), sodium triacetoxyborohydride, lithium borohydride (LiBH₄), lithium aluminum hydride (LiAlH₄), (i-Bu₂AlH)₂, L-selectride, K-selectride, SnCl₂, phenylsilane, Et₃SiH, and mixtures thereof, with sodium cyanoborohydride (NaCNBH₃) being preferred. In the present invention, the reducing agent may be added in an amount of 1 to 5 equivalents, preferably 2 to 5 equivalents, relative to the ligand, without being limited thereto.

In the present invention, the reaction of the trifluoroacetic acid of Formula 1 with the ligand containing the amine group may be performed at 10 to 150°C, preferably 25 to 150°C, more preferably 100 to 150°C, most preferably 120°C, without being limited thereto.

In the present invention, the reaction of the trifluoroacetic acid of Formula 1 with the ligand containing the amine group may be performed for 5 to 120 minutes, preferably 15 to 60 minutes, more preferably 20 to 40 minutes, without being limited thereto.

Non-limiting examples of ligands substituted with a trifluoroethyl group according to the method of the present invention include, but are not limited to, compounds having the structures below:

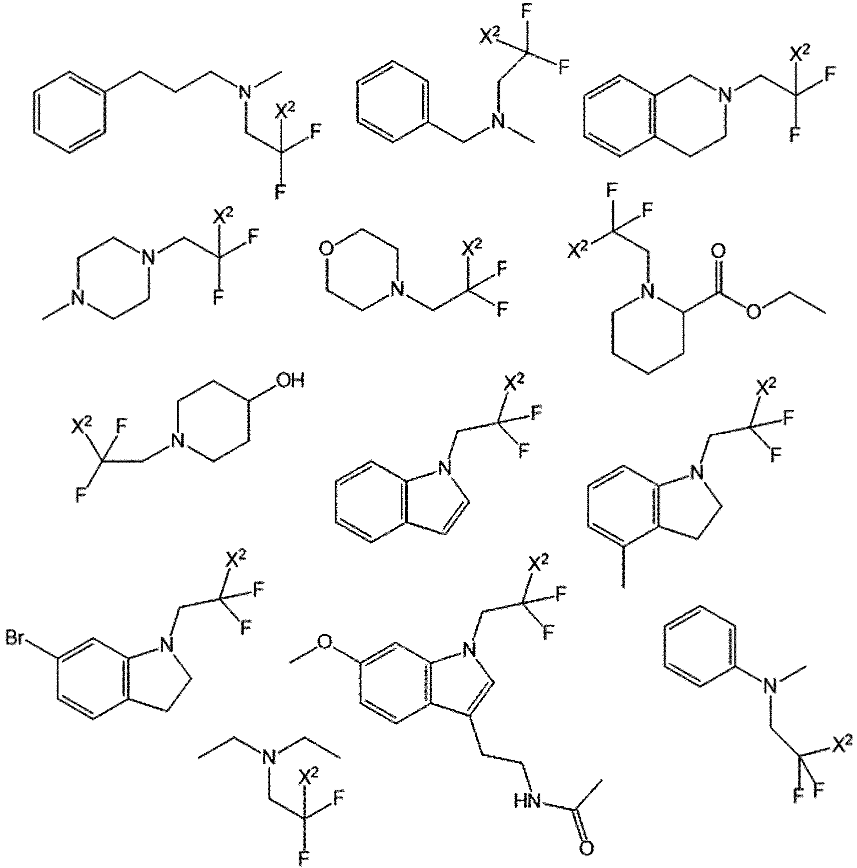

In the formulas above, X² is ¹⁸F or ¹⁹F.

According to the method of the present invention, a trifluoroalkyl group, particularly a trifluoromethyl group or a trifluoroethyl group, may be transferred to a target ligand, and when [¹⁸F] fluoride ion is used as the fluoride ion, a ligand having a fluorine-18-labeled trifluoroalkyl group introduced therein may be produced. The ligand having the -CF₂[¹⁸F] group, when administered *in vivo,* has excellent *in vivo* stability, and also has excellent lipophilicity and cell penetration ability.

### Mode for Invention

Hereinafter, the present invention will be described in more detail through examples. These examples are only intended to explain the present invention in more detail, and it will be apparent to those skilled in the art that the scope of the present invention according to the subject matter of the present invention is not limited by these examples.

### Examples

### [Preparation Example 11 Synthesis of Copper Complex Compound

To synthesize trifluoroacetic acid, a copper complex compound as a reactant was first synthesized. Specifically, 1.2 mmol of NaOtBu was dissolved in 5 mL of THF in a glove box, and 1.0 mmol of CuCl dissolved in 5 mL of THF was added thereto, followed by stirring at room temperature for 90 minutes. The reaction mixture was passed through celite, and 2.0 mmol of 2,2'-bipyridine (Bpy) dissolved in 10 mL of THF was added to the resulting filtrate, followed by stirring at room temperature for 10 minutes. 1.0 mmol of chlorodifluoroacetic acid dissolved in 3 mL of THF was added thereto dropwise, followed by stirring at room temperature for 10 minutes. The solvent of the reaction mixture was removed *in vacuo,* the remaining solid material was washed with diethyl ether, and the remaining solvent was completely removed *in vacuo.* As copper complex compound, [(Bpy)₂Cu][CF₂ClCO₂] represented by Formula 31 below was synthesized, and its synthesis was 70%.

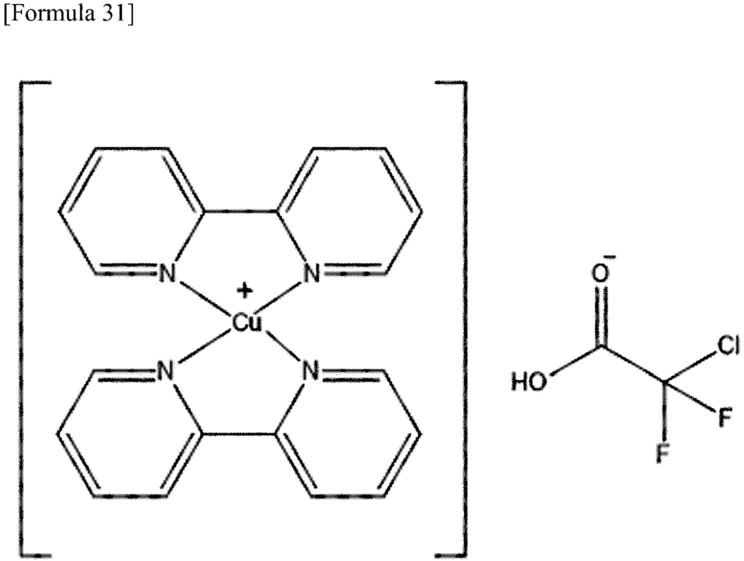

### [Preparation Example 2] Synthesis of Copper Complex Compound

[(Phen)₂Cu][CF₂ClCO₂] represented by Formula 32 below was synthesized in a yield of 73% in the same manner as in Preparation Example 1, except that 1,10-phenanthroline (Phen) was used instead of 2,2'-bipyridine as the ligand for producing the copper complex.

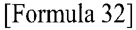

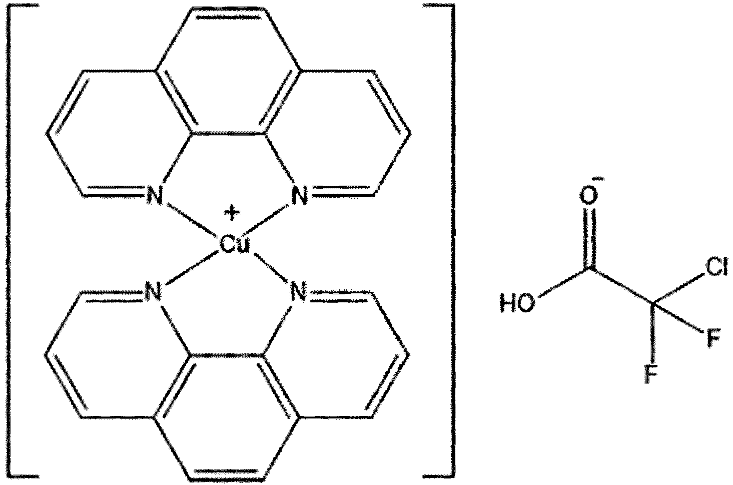

### [Preparation Example 31 Synthesis of Copper Complex Compound

[(TMPhen)₂Cu][CF₂ClCO₂] represented by Formula 33 below was synthesized in a yield of 68% in the same manner as in Preparation Example 1, except that 3,4,7,8-tetramethyl-1,10-phenanthroline was used instead of 2,2'-bipyridine as the ligand for producing the copper complex.

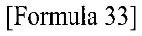

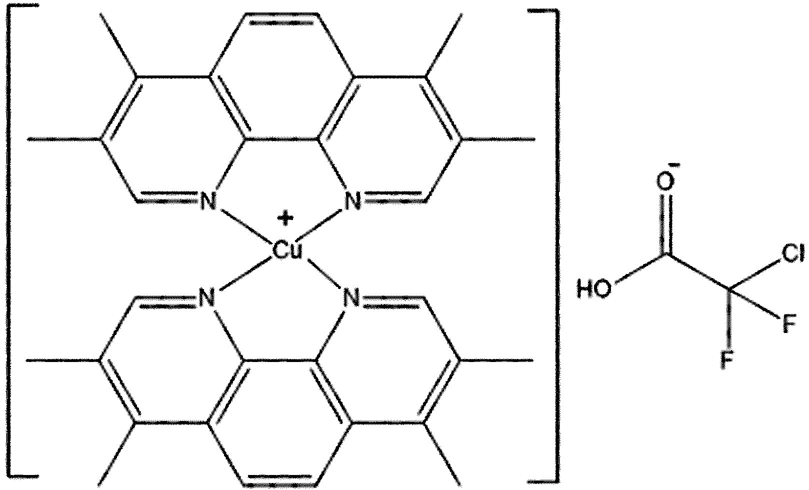

### [Experimental Example 1] Comparison of Synthesis Yields of Trifluoroacetic Acid (TFA) Depending on Reactants

Chlorodifluoroacetic acid or the copper complex compound (0.02 mmol) synthesized in each of Preparation Examples 1 to 3, Cs₂CO₃ (0.02 mmol) as a base, and CsF (0.02 mmol) as a fluoride ion source were dissolved in DMF, followed by stirring at 75°C for 1 hour, thereby synthesizing trifluoroacetic acid.

However, in order to compare the synthesis yield of trifluoroacetic acid according to the type of reactant used in the reaction or the use or non-use of the base, after completion of the reaction, the reaction mixture was sufficiently cooled to room temperature, and then the amount of trifluoroacetic acid produced was measured by ¹⁹F-NMR. The results are shown in Table 1 below.

**[Table 1]**

| | Reagent | Base | Synthesis yield (%) |
|---|---|---|---|
| Comparative Example 1 | CF₂ClCOOH | None | NR |
| Example 1 | CF₂ClCOOH | Cs₂CO₃ | 1.6 ± 0.4 |
| Example 2 | Preparation Example 1: [(BPy)₂Cu][CF₂ClCO₂] | Cs₂CO₃ | 6.6 ± 0.7 |
| Example 3 | Preparation Example 2: [(Phen)₂Cu] [CF₂ClCO₂] | Cs₂CO₃ | 7.5 ± 1.1 |
| Example 4 | Preparation Example 3: [(TMPhen)₂Cu] [CF₂ClCO₂] | Cs₂CO₃ | 10.3 ± 1.2 |

As can be seen in Table 1 above, when only chlorodifluoroacetic acid was used, trifluoroacetic acid was not produced, but when the base (Cs₂CO₃) was added, trifluoroacetic acid was synthesized. In addition, when the copper complex compound synthesized in each of Preparation Examples 1 to 3 as a reactant was reacted with the base (Cs₂CO₃), trifluoroacetic acid was synthesized. It could be seen that the synthesis yield of trifluoroacetic acid was the highest when [(TMPhen)₂Cu][CF₂ClCO₂] synthesized in Preparation Example 3 among the copper complex compounds was used.

### [Experimental Example 2] Comparison of Synthesis Yields of Trifluoroacetic Acid (TFA) Depending on Reaction Time and Equivalent Weight of Fluorine Added

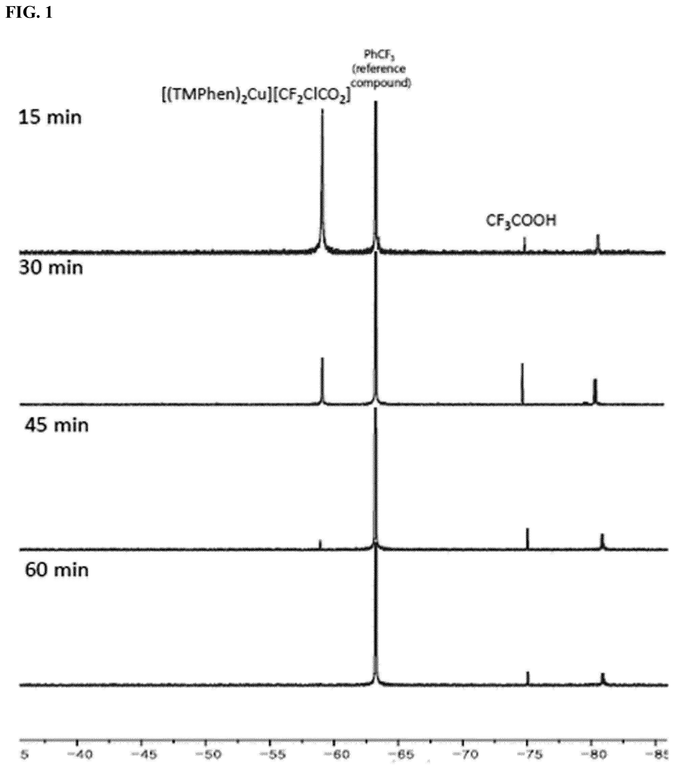

An experiment was conducted to determine the degree of trifluoroacetic acid synthesis depending on the fluorine equivalent weight and reaction time before labeling with fluorine-18. Since the amount of fluorine-18 is extremely small compared to that of fluorine-19, the amount of trifluoroacetic acid produced was compared depending on the equivalent weight of fluorine-19 and the reaction time. [(TMPhen)₂Cu][CF₂ClCO₂] (0.02 mmol), a copper complex compound that showed the highest synthesis yield in Experimental Example 1 above, and Cs₂CO₃ (0.02 mmol) were placed in a reaction vessel, and CsF as a fluorine source was dissolved in DMF in three different amounts (0.004, 0.02, and 0.04 mmol) and added thereto, followed by stirring at 75°C for 1 hour. After the reaction vessel was sufficiently cooled to room temperature at each reaction time (15, 30, 45, or 60 min), the amount of trifluoroacetic acid produced was measured by ¹⁹F-NMR. The results are shown in FIG. 1 and Table 2 below.

**[Table 2]**

| | CsF (eq.) | TFA synthesis yield (%) | | | |
|---|---|---|---|---|---|
| | | 15 min | 30 min | 45 min | 60 min |
| Example 5 | 1 | 11 | 35 | 20 | 10 |
| Example 6 | 2 | 7 | 15 | 8 | 1 |
| Example 7 | 0.2 | 3 | 16 | 13 | 4 |

As shown in Table 2 above and FIG. 1, it could be confirmed that, regardless of the amount of CsF added, trifluoroacetic acid was produced in the largest amount 30 minutes after the start of the reaction and the amount produced decreased thereafter. In addition, it could be confirmed that trifluoroacetic acid was produced even when CsF as a fluorine source was added in a small amount of 0.2 equivalents, and that the amount of trifluoroacetic acid produced was the largest when CsF was added in an amount of 1 equivalent.

### [Experimental Example 3] Evaluation of Efficiency of N-Trifluoroethylation of Ligand Depending on Reducing Agent and Reaction Temperature

The following experiment was conducted to confirm the optimal reaction conditions for N-trifluoroethylation by in-situ reaction using trifluoroacetic acid synthesized in the previous experiment.

The copper complex compound [(TMPhen)₂Cu][CF₂ClCO₂] (3 eq.), Cs₂CO₃ (3 eq.), and CsF (3 eq.) were placed in a reaction vessel, dissolved in DMF, and then stirred at 75°C for 30 minutes. After cooling the reaction mixture to room temperature, a model compound (1 eq.) of Formula 34 containing a secondary amine group and a reducing agent were added thereto in the types and amounts shown in Table 3 below, followed by stirring for an additional 30 minutes at room temperature or 125°C. After the reaction mixture was sufficiently cooled to room temperature, the production yield of the final product was measured by ¹⁹F-NMR, and the results are shown in Table 3 below.

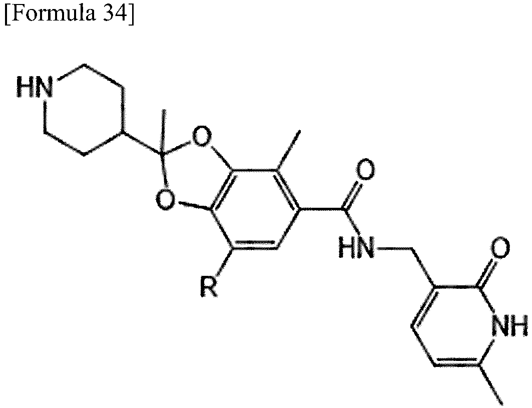

**[Table 3]**

| | Reducing agent | Equivalent weight of reducing agent | Reaction temperature (°C) | Production yield (%) |
|---|---|---|---|---|
| Example 8 | NaBH₄ | 2 eq. | 120 | 26.5 ± 4.9 |
| Example 9 | NaBH₄ | 5 eq. | 120 | 11.5 ± 3.0 |
| Example 10^{a.} | NaCNBH₃ | 2 eq. | 25 | 8.7 ± 1.2 |
| Example 11^{a.} | NaCNBH₃ | 2 eq. | 120 | 3.8 ± 0.7 |
| Example 12 | NaCNBH₃ | 1 eq. | 120 | 20.1 ± 2.6 |
| Example 13 | NaCNBH₃ | 2 eq. | 120 | 42.9 ± 1.7 |
| Example 14 | NaCNBH₃ | 5 eq. | 120 | 15.3 ± 2.3 |
| ^{a.} HCl (0.1 eq.) added | | | | |

As shown in Table 3 above, when NaBH₄ was used as a reducing agent in an amount of 2 equivalents, the efficiency of trifluoroethylation was approximately 26.5%, but when the equivalent weight increased, the efficiency was observed to show a slight decreasing tendency rather than an increase. Meanwhile, it could be seen that, when NaCNBH₃ was used as a reducing agent, the efficiency of trifluoroethylation increased to 40%. However, it could be confirmed that, when the reaction was carried out under acidic conditions as in Examples 10 and 11 or at room temperature, the efficiency of trifluoroethylation decreased rather than increased.

### [Experimental Example 4] Synthesis of Fluorine-18-Labeled Trifluoroacetic Acid

[¹⁸F]fluoride was obtained through the 180(p,n)¹⁸F reaction from a cyclotron, and then [¹⁸F]fluoride was separated from QMA Sep-pak using an acetonitrile solution containing K_{2,2,2} and K₂CO₃ dissolved therein and heated to obtain K¹⁸F/K_{2,2,2}. The [(TMPhen)₂Cu][CF₂ClCO₂] copper complex (10 mg) obtained in Preparation Example 3 and Cs₂CO₃ (9.5 mg) were added to a reaction vessel containing K¹⁸F/K_{2,2,2}, dissolved in DMF (0.3 mL), and, after adding a stirring bar, stirred at 70°C for 20 minutes. After the reaction time was over, the reaction vessel was sufficiently cooled to room temperature, and then HPLC was performed using an ion exchange column under the conditions of 5 mM H₂SO₄ 100%, 0.5 mL/min, 210 nm to confirm the reaction mixture product.

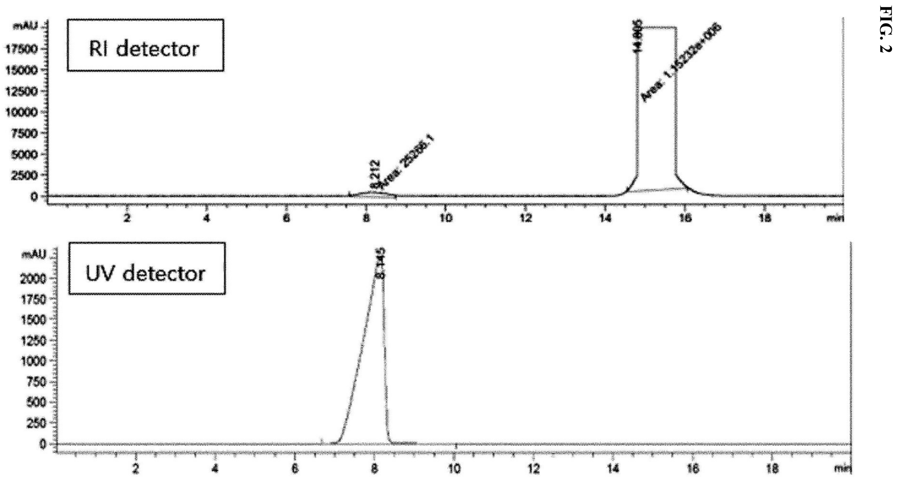

In the HPLC results by the RI detector as shown in FIG. 2, a peak was observed at approximately 8.2 minutes. In the HPLC results by the UV detector, a ¹⁹F-TFA peak was observed at approximately 8.1 minutes, and thus through comparison of the retention times, it could be seen that the peak at 8.2 minutes confirmed in the RI HPLC results was due to the ¹⁸F-TFA (RI detector). In the same way, it could be confirmed that unreacted remaining fluorine-18 was observed at 14.8 minutes.

### [Experimental Example 5] Separation and Formulation of Fluorine-18-Labeled Trifluoroacetic Acid from Reaction Mixture

### 1. Separation of ¹⁸F-TFA from Reaction Mixture

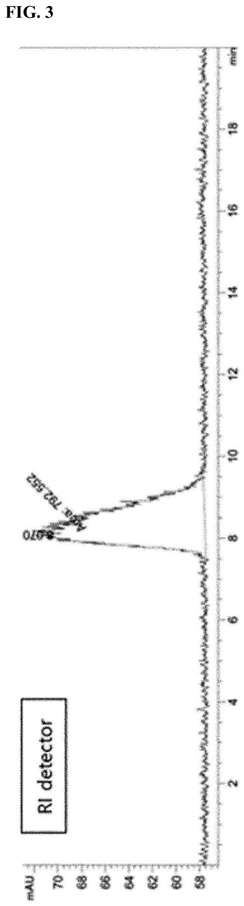

From Experimental Example 4, it could be confirmed that fluorine-18-labeled trifluoroacetic acid (¹⁸F-TFA) was produced through the reaction of the copper complex compound with [¹⁸F] fluoride ion, but unreacted fluorine-18 was present. Therefore, in order to separate only ¹⁸F-TFA from the reaction mixture, a Sep-Pak alumina N plus cartridge (Sep-Pak Alumina N plus^{™}, manufactured by Waters Co.) was used. The Sep-Pak cartridge was pre-conditioned by sequentially passing 5 mL of ethanol and 20 mL of water therethrough. The reaction mixture obtained in Experimental Example 4 was diluted by adding 20 mL of water and then passed through the Sep-Pak cartridge. Thereafter, the Sep-Pak cartridge was washed by passing 10 mL of water therethrough. To confirm whether unreacted fluorine-18 was removed from the reaction mixture and only ¹⁸F-TFA was separated, the radioactivities of the reaction mixture before washing, the Sep-Pak cartridge after washing, and the waste that passed through the Sep-Pak cartridge were measured using a dose calibrator, and the results are shown in Table 4 below. In addition, HPLC analysis was performed on the waste that passed through the Sep-pak cartridge, and the results are shown in FIG. 3.

**[Table 4]**

| | Reaction mixture radioactivity (µCi) | Sep-pak radioactivity (µCi) | Waste radioactivity (µCi) |
|---|---|---|---|
| Alumina N plus | 416.8 | 362.4 | 46.2 |

As shown in Table 4 above, the radioactivity in the Sep-pak to which the reaction mixture was adsorbed was 362.4 µCi, and the radioactivity in the waste that passed through the Sep-pak was 46.2 µCi. In addition, referring to FIG. 3 showing the result of HPLC analysis on the waste, the ¹⁸F-TFA peak was observed at approximately 8.0 minutes, but the fluorine-18 peak could not be confirmed. This suggests that, when the reaction mixture was passed through the Sep-pak cartridge, fluorine-18 was adsorbed on the Sep-pak and remained, and ¹⁸F-TFA was eluted and separated.

### 2. Formulation of ¹⁸F-TFA

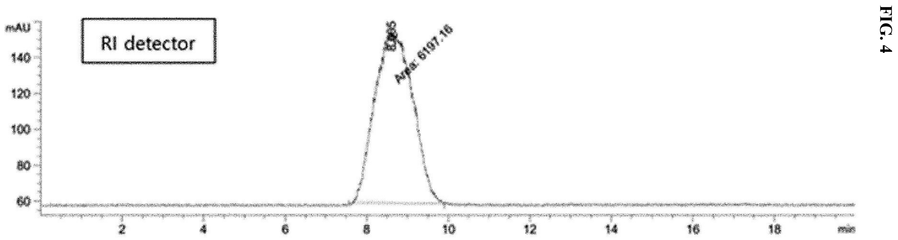

Since the previously separated ¹⁸F-TFA remains diluted in 20 mL of water, a formulation process is required to change it into an appropriate amount of a required solution for use in the trifluoroethylation of the ligand. For formulation, an ion exchange Sep-pak was used to adsorb ¹⁸F-TFA onto the Sep-pak, and then an experiment was conducted to determine whether it could be eluted as the required solution. An Oasis^{®} WAX Plus^{™} (manufactured by Nihon Waters K.K. product) cartridge was pre-conditioned by sequentially passing 5 mL of ethanol and 20 mL of water therethrough. Thereafter, the waste containing the previously separated ¹⁸F-TFA and 20 mL of water was passed through the WAX Sep-pak. The amount of radioactivity adsorbed on the Sep-pak cartridge was measured, and the radioactivity for the waste obtained after elution with saline (1 mL) was measured. The results are shown in Table 5 below. In addition, HPLC analysis was performed on the waste, and the results are shown in FIG. 4.

**[Table 5]**

| Process | Radioactivity (µCi) |
|---|---|
| First waste from reaction product | 46.2 |
| Adsorption on WAX Sep-pak | 36.9 |
| Second waste obtained after elution with saline | 22.1 |

As shown in Table 5 above, it could be confirmed that most of the ¹⁸F-TFA contained in the first waste from the reaction product was adsorbed on the WAX Sep-pak cartridge and eluted with saline. Referring to FIG. 4 showing the results of HPLC analysis on the second waste obtained after elution with saline, the ¹⁸F-TFA peak was observed at approximately 8.0 minutes. Thereby, it could be seen that ¹⁸F-TFA synthesized according to the present invention can be separated and purified using a Sep-pak cartridge containing alumina, and can be formulated into a desired formulation using a WAX cartridge.

### [Experimental Example 6] Evaluation of N-Trifluoroethylation of Ligand

The reaction mixture containing ¹⁸F-TFA obtained in Experimental Example 4 was cooled to room temperature, and then a ligand containing a primary amine group (1 eq.) and NaCNBH₃ (5 eq.) were added thereto, followed by stirring at 125°C for an additional 30 minutes. After the reaction mixture was sufficiently cooled to room temperature, the product was separated by HPLC, and the yield was measured by the amount of radioactivity. As a result, a radiochemical yield of approximately 17% and a specific radioactivity of 2.12 GBq/µmol could be obtained. The obtained product was coinjected into HPLC with a ligand containing fluorine-19 prepared in advance, and the compound was identified by comparing the retention times. However, HPLC analysis was performed using an Xterra C18 column for 20 minutes at a wavelength of 254 nm with a flow rate of 3 mL/min under the conditions of 0.1% formic acid/CH₃CN: 0.1% formic acid/D.W. = 35:65.

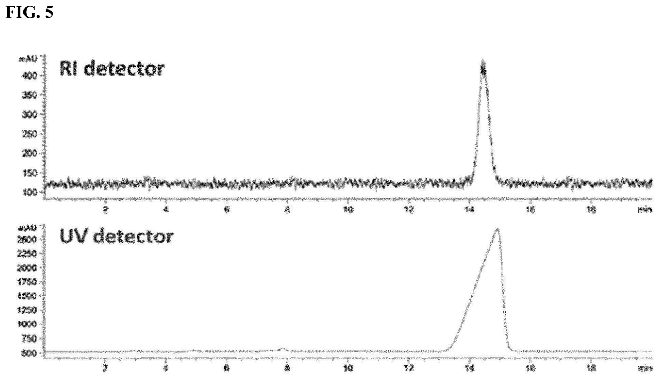

As shown in FIG. 5, it could be seen that [¹⁸F]N-trifluoroethylation of a ligand containing a primary amine group was achieved using ¹⁸F-TFA synthesized according to the present invention.

Through the above experiments, it could be seen that, according to the method of the present invention, fluorine-18-labeled trifluoroacetic acid can be easily synthesized in high yield, and furthermore, it can be separated and formulated. In addition, the use of the fluorine-18-labeled trifluoroacetic acid synthesized in the present invention enables [¹⁸F]trifluoroethylation of a ligand containing an amine group in the structure, and the ligand into which the [¹⁸F]trifluoroethyl group has been introduced in this way has the advantage of excellent stability when administered *in vivo* and also excellent cell penetration ability.

### [Experimental Example 7] Evaluation of Efficiency of Trifluoromethylation of Ligand Depending on Reaction Conditions

The following experiment was conducted to confirm the optimal reaction conditions for performing N-trifluoromethylation using the [(TMPhen)₂Cu][CF₂ClCO₂] copper complex compound synthesized in Preparation Example 3 above.

### 1. Selection of optimal reaction conditions for N-trifluoromethylation depending on fluoride ion source

The model compound 1-iodo-4-nitrobenzene (1 eq.), the copper complex compound [(TMPhen)₂Cu][CF₂ClCO₂] (n eq.), Cs₂CO₃ (n eq.), and CsF (3 eq.) or K¹⁸F/K_{2.2.2} as a fluoride ion source were added to a reaction vessel and then dissolved in DMF. The reaction was carried out at 75°C or 150°C. When CsF was used as a fluoride ion source, the reaction was carried out with stirring for 1 hour, and when K¹⁸F/K_{2,2,2} was used as a fluoride ion source, the reaction was carried out with stirring for 20 minutes. After the reaction mixture was sufficiently cooled to room temperature, the yield of the final product was measured by ¹⁹F-NMR, and the results are shown in Table 6 below. When the radioisotope was used, the radiochemical yield was measured by Radio-TLC, and the results are shown in Table 7.

**[Table 6]**

| | Copper complex compound | Fluoride ion source | Base (eq.) | Reaction temperature (°C) | Yield (%) |
|---|---|---|---|---|---|
| Example 15 | 2 eq. | CsF | Cs₂CO₃ (3 eq.) | 75 | 81.5 ± 0.7 |
| Example 16 | 1.5 eq. | CsF | Cs₂CO₃ (1 eq.) | 75 | 85.0 ± 1.4 |
| Example 17 | 1 eq. | CsF | Cs₂CO₃ (1 eq.) | 75 | 57.5 ± 0.7 |
| Example 18 | 1 eq. | CsF | Cs₂CO₃ (3 eq.) | 150 | 23.5 ± 4.9 |

**[Table 7]**

| | Copper complex compound | Fluoride ion source | Base (eq.) | Reaction temperature (°C) | Radiochemic al yield (%) |
|---|---|---|---|---|---|
| Example 19 | 2 eq | K¹⁸F/K_{2,2,2} | Cs₂CO₃ (3 eq) | 75 | 4.8 ± 3.6 |
| Example 20 | 1.5 eq | K¹⁸F/K_{2,2,2} | Cs₂CO₃ (1 eq) | 75 | 2.2 ±1.9 |
| Example 22 | 1 eq | K¹⁸F/K_{2,2,2} | Cs₂CO₃ (3 eq) | 150 | 50.3± 2.3 |

As shown in Table 6 above, the reaction using CsF, i.e., fluorine-19, as a fluoride ion source had the best reaction yield at 75°C, and the highest reaction yield was obtained when 1.5 equivalents of the copper complex compound and 1 equivalent of the base were used. However, it could be confirmed that the reaction yield decreased when the reaction temperature was increased to 150°C. As shown in Table 7 above, it could be confirmed that, in the reaction using K¹⁸F/K2_{,2,2,2}, i.e., fluorine-18, as a fluoride ion source, the reaction yield and radiochemical yield increased significantly when the reaction temperature was increased to 150°C compared to when it was set to 75°C. Therefore, in the reaction using fluorine-18, the conditions of using 1 equivalent of copper complex compound, using 3 equivalents of base, and setting the reaction temperature to 150°C were selected as the optimal reaction conditions.

### 2. Checking for Labeling with Fluorine-18

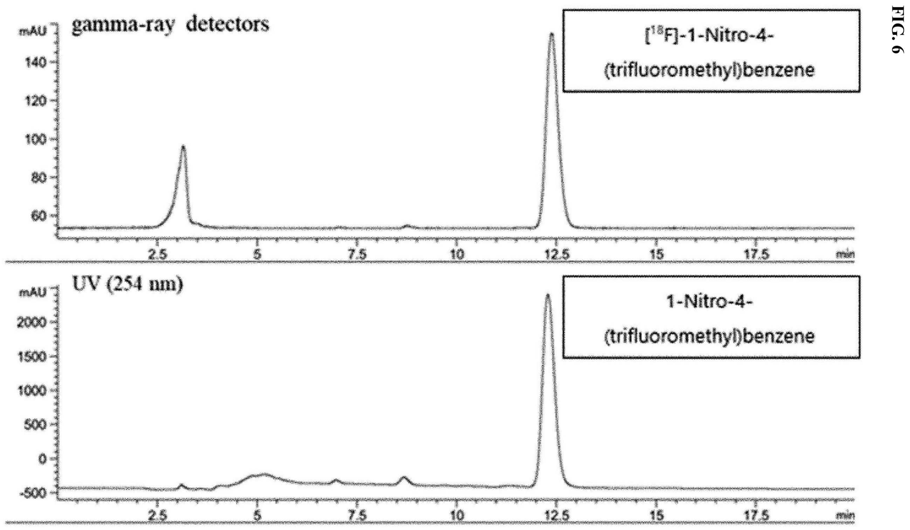

To confirm whether the compound obtained using fluorine-18 under the optimal reaction conditions was actually labeled with fluorine-18, HPLC analysis was performed, and the retention time of the compound was compared with that of the compound into which ¹⁹F has been introduced. However, the HPLC analysis conditions were as follows. The analysis results are shown in FIG. 6.
System: Agilent Separation Products System with gamma-ray detectors and UV (254 nm)
Semi-preparative Column: Xterra, RP-18 C18; 4.6 x 250 mm, 10 µm
Eluent: 50% CH₃CN/H₂O
Flow rate: 3 mL/min

As shown in FIG. 6, it could be confirmed that the iodo group of 1-iodo-4-nitrobenzene was substituted with a fluorine-18-labeled trifluoromethyl group (-CF₂¹⁸F) through the above reaction.

### [Experimental Example 8] Evaluation of Efficiency of Trifluoromethylation in Various Ligands

The following experiments were conducted on ligands having various functional groups to confirm whether trifluoromethylation was possible by reacting a copper complex compound with [¹⁸F]fluoride ion or [¹⁹F]fluoride ion.

### 1. Evaluation of Yield of Trifluoromethylation of Each Ligand

Each of ligands (1 eq.) substituted with various functional groups while containing an iodo-substituted aryl group or heteroaryl group as shown in Table 7 below, the copper complex compound [(TMPhen)₂Cu][CF₂ClCO₂] (1.5 eq.), Cs₂CO₃ (1 eq.), and CsF (3 eq.) as a fluoride ion source were added to a reaction vessel, dissolved in DMF, and stirred at 75°C for 1 hour. Next, the reaction vessel was cooled to room temperature and the yield of the final product was measured by ¹⁹F-NMR. The results are shown in Table 8 below.

**[Table 8]**

| | Reactant | Product | Yield (%) |
|---|---|---|---|
| Example 23 | 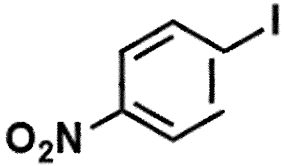 | 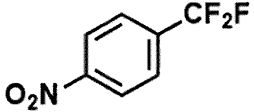 | 85 |
| Example 24 | 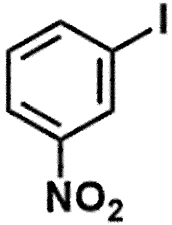 | 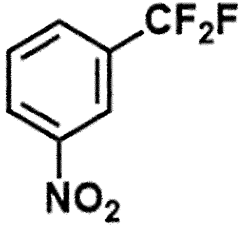 | 78 |
| Example 25 | 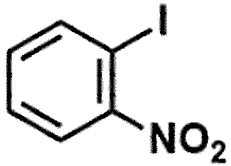 | 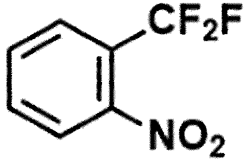 | 60 |
| Example 26 | 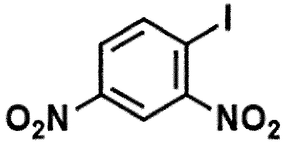 | 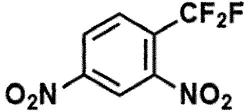 | 68 |
| Example 27 | 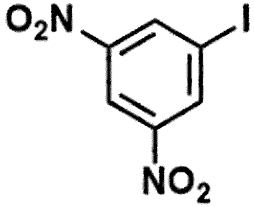 | 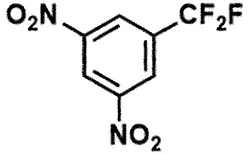 | 51 |
| Example 28 | 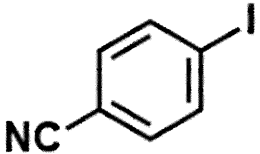 | 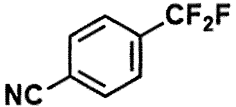 | 59 |
| Example 29 | 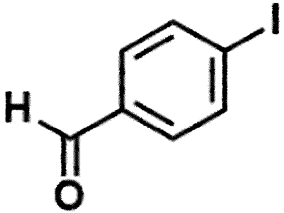 | 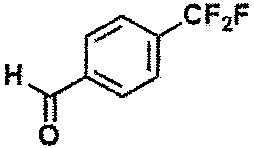 | 28 |
| Example 30 | 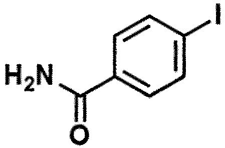 | 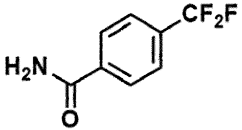 | 20 |
| Example 31 | 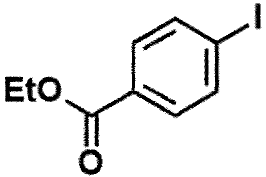 | 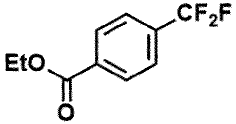 | 30 |
| Example 32 | 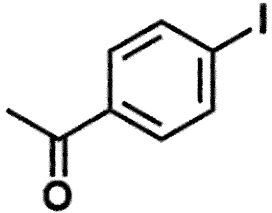 | 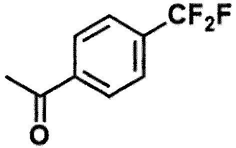 | 36 |
| Example 33 | 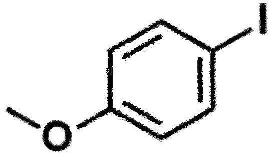 | 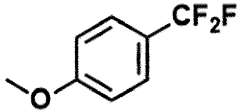 | 26 |
| Example 34 | 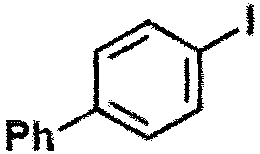 | 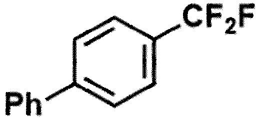 | 27 |
| Example 35 | 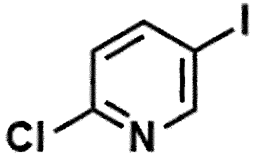 | 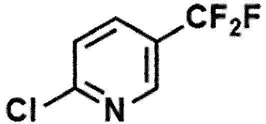 | 31 |
| Example 36 | 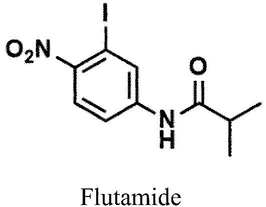 | 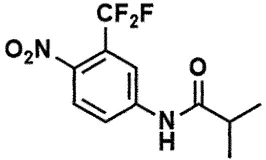 | 47 |

As shown in Table 8 above, it could be confirmed that trifluoromethylation of various ligands containing an iodo-substituted aryl or heteroaryl structure was achieved. In particular, it could be seen that even flutamide (Example 36), which is used as a prostate cancer therapeutic agent, was trifluoromethylated.

### 2. Evaluation of Yield of Fluorine-18- Labeled Trifluoromethylation of Each Ligand

Each ligand (1 eq.) shown in Table 7 above, the copper complex compounds [(TMPhen)₂Cu][CF₂ClCO₂] (1 eq.), Cs₂CO₃ (3 eq.), and K¹⁸F/K_{2,2,2} as a fluoride ion source were added to a reaction vessel, dissolved in DMF, and then stirred at 150°C for 20 minutes. Thereafter, the reaction vessel was cooled to room temperature, and the yield of the final product was measured by ¹⁸F-NMR. The results are shown in Table 9 below.

**[Table 9]**

| | Reactant | Product | Yield (%) |
|---|---|---|---|
| Example 37 | 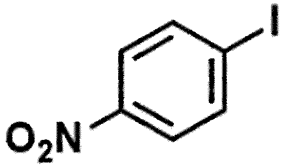 | 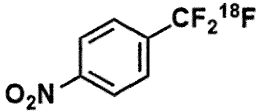 | *50* |
| Example 38 | 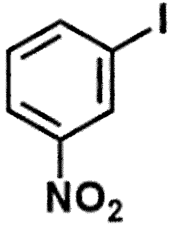 | 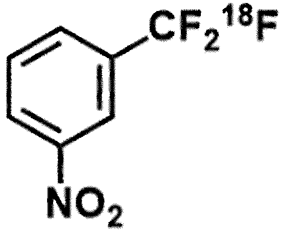 | 55 |
| Example 39 | 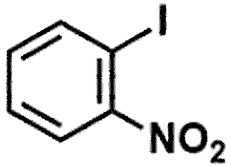 | 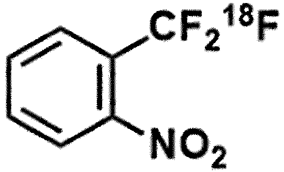 | 46 |
| Example 40 | 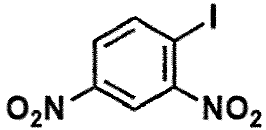 | 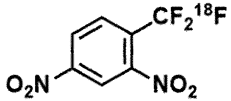 | 16 |
| Example 41 | 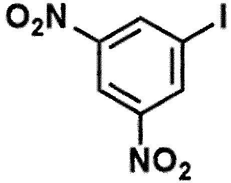 | 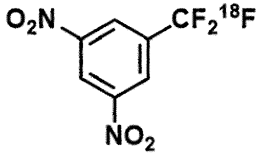 | 21 |
| Example 42 | 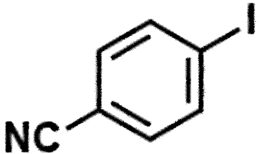 | 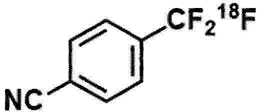 | 31 |
| Example 43 | 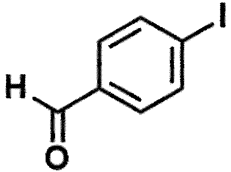 | 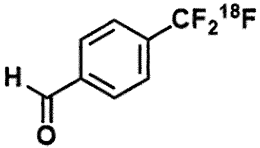 | 25 |
| Example 44 | 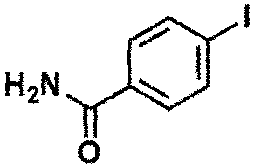 | 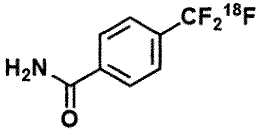 | 69 |
| Example 45 | 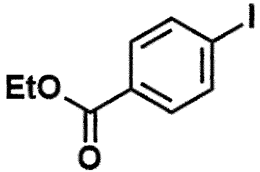 | 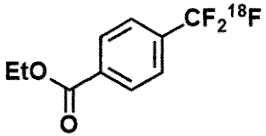 | 32 |
| Example 46 | 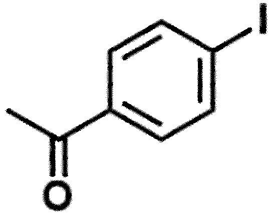 | 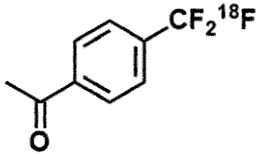 | 15 |
| Example 47 | 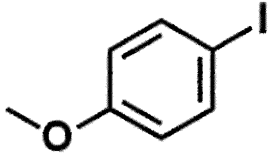 | 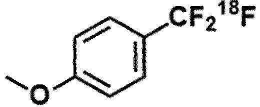 | 36 |
| Example 48 | 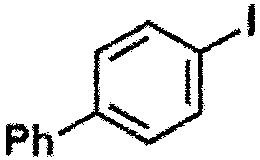 | 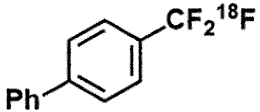 | 24 |
| Example 49 | 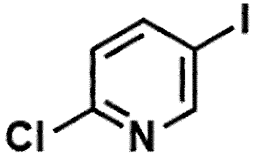 | 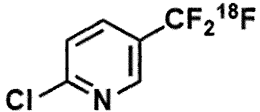 | 21 |
| Example 50 | 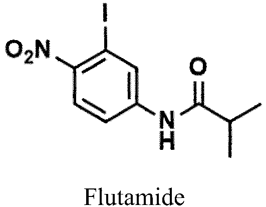 | 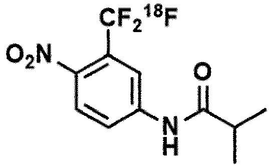 | 41 |

As shown in Table 9 above, it could be confirmed that fluorine-18 labeled-trifluoromethylation of various ligands containing an iodo-substituted aryl or heteroaryl structure was achieved, and that fluorine-18-labeled trifluoromethylation of flutamide (Example 50), which is used as a prostate cancer therapeutic agent, was also achieved in high yield.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a building block that may be utilized for trifluoroalkylation in the production of a radioactive fluorine-18-labeled radiopharmaceutical for medical diagnosis, a method for producing the same, and a method of trifluoroalkylating a ligand using the same.

## Claims

1. A trifluoroalkylation method comprising a step of reacting a copper complex compound of Formula 1 below with a ligand in the presence of a fluoride ion, thereby trifluoroalkylating the ligand: 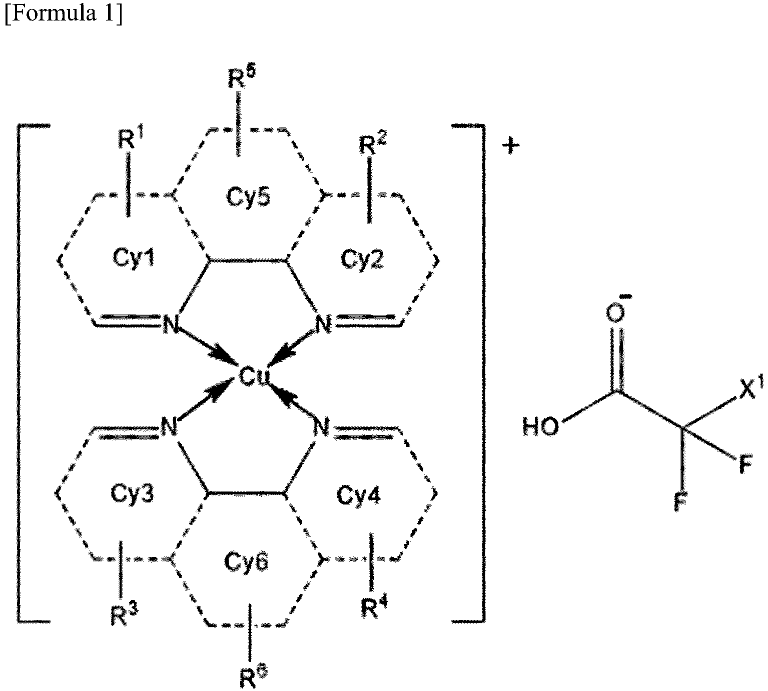 wherein
Cy1 to Cy4 are each independently a 4- to 8-membered heteroaryl or heterocycloalkenyl ring,
Cy5 and Cy6 are each independently a 0- to 8-membered cycloalkyl, cycloalkenyl, aryl, heteroaryl, heterocycloalkyl or heterocycloalkenyl ring,
R¹ to R⁶ are each independently hydrogen, a halogen, nitro, a C₁-C₆ alkyl group, or a C₆-C₁₄ aryl group, and are mono-substituents or the same or different multi-substituents, and
X¹ is a halogen other than a fluoro group.

2. The trifluoroalkylation method of claim 1, wherein the fluoride ion is [¹⁸F]fluoride ion or [¹⁹F]fluoride ion.

3. The trifluoroalkylation method of claim 1, wherein the fluoride ion is added in an amount of 0.2 to 4 equivalents relative to the copper complex compound.

4. The trifluoroalkylation method of claim 1, wherein the reaction is performed in the presence of a base.

5. The trifluoroalkylation method of claim 4, wherein the base is NaH, LiH, KH, K₂CO₃, Na₂CO₃, Tl₂CO₃, Cs₂CO₃, K(OtBu), Li(OtBu), Na(OtBu), K(OAr), Na(OAr), triethylamine, or a mixture thereof.

6. The trifluoroalkylation method of claim 4, wherein the base is added in an amount of 0.5 to 4 equivalents relative to the copper complex compound of Formula 1.

7. The trifluoroalkylation method of claim 1, wherein the ligand comprises an aryl or heteroaryl structure substituted with a halogen, a boronic acid group, or a boronic acid pinacol ester group, and the halogen, boronic acid group, or boronic acid pinacol ester group in the ligand is substituted with a trifluoromethyl group in the step.

8. The trifluoroalkylation method of claim 7, wherein the ligand is a compound represented by Formula 15 below: 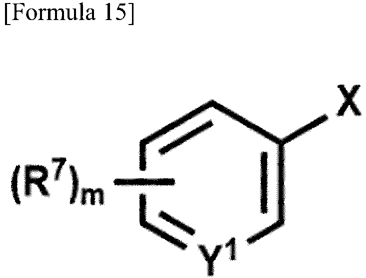 wherein
X is a halogen, a boronic acid group or a boronic acid pinacol ester group,
Y¹ is N or C(R⁸),
m is an integer ranging from 0 to 4,
R⁷ is a halogen, nitro, a cyano group, a C₁-C₆ alkyl group, a C₆-C₁₄ aryl group, a C₁-C₆ alkoxy group, -C(=O)-R⁹, or -NR¹⁰C(=O)-R¹¹, and when there are a plurality of R⁷s, they are the same as or different from each other,
R⁸ is hydrogen or a C₁-C₆ alkyl group,
R⁹ is hydrogen, an amine group (-NH₂), a C₁-C₆ alkyl group, or a C₁-C₆ alkoxy group, and
R¹⁰ and R¹¹ are each independently hydrogen or a C₁-C₆ alkyl group.

9. The trifluoroalkylation method of claim 7, wherein the ligand is selected from among compounds having the following structures: 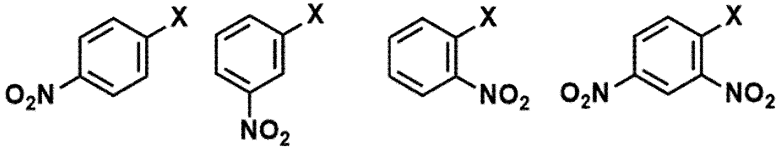 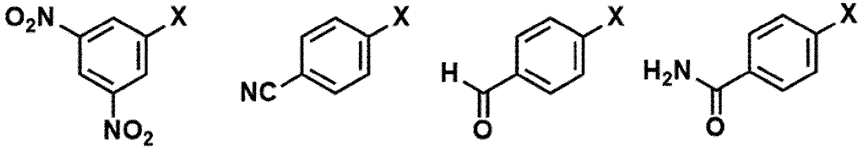 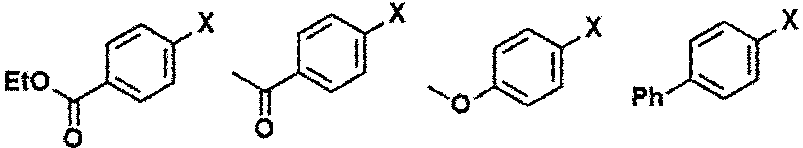 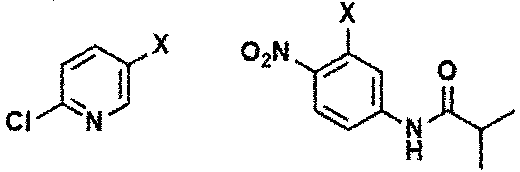 wherein X is a halogen, a boronic acid group, or a boronic acid pinacol ester group.

10. The trifluoroalkylation method of claim 7, wherein the fluoride ion is added in an amount of 0.2 to 4 equivalents relative to the copper complex compound, and the reaction is performed at a temperature of 50 to 200°C for 5 to 120 minutes.

11. The trifluoroalkylation method of claim 7, wherein the reaction is performed in the presence of the base in an amount of 0.5 to 4 equivalents relative to the copper complex compound.

12. The trifluoroalkylation method of claim 10, wherein the fluoride ion is [¹⁸F]fluoride ion, and the reaction is performed at 100 to 200°C for 10 to 30 minutes.

13. The trifluoroalkylation method of claim 10, wherein the fluoride ion is [¹⁹F]fluoride ion, and the reaction is performed at 60 to 80°C for 30 to 100 minutes.

14. The trifluoroalkylation method of claim 1, wherein the ligand comprises a primary amine group or a secondary amine group, and hydrogen (H) of the amine group in the ligand is substituted with a trifluoroethyl group in the step.

15. The trifluoroalkylation method of claim 14, wherein the ligand is selected from the group consisting of N-methyl-3-phenylpropan-1-amine, N-methylbenzylamine, N,N-diethyl-amine, 1,2,3,4-tetrahydroisoquinoline, 1-methyl-4-piperazine, 4-morpholine, ethyl piperidine-2-carboxylate, piperidin-4-ol, N-(2-(6-methoxy)indolin-3-yl)ethyl)acetamide, indole, 6-bromoindoline, 4-methylindoline, and N-methylbenzenamine.

16. The trifluoroalkylation method of claim 14, wherein the step comprises a step of reacting the copper complex compound of Formula 1 with a fluoride ion source to produce a trifluoroacetic acid represented by Formula 14 below; and a step of reacting the trifluoroacetic acid with the ligand: 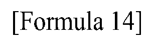 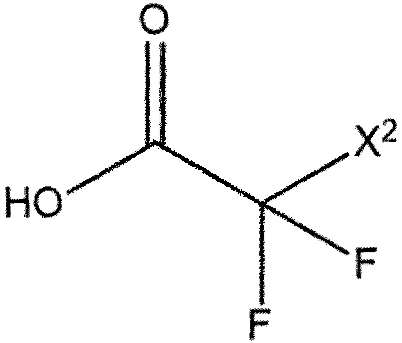 wherein X² is ¹⁸F or ¹⁹F.

17. The trifluoroalkylation method of claim 14, wherein the reaction of the copper complex compound with the fluoride ion is performed in the presence of the base added in an amount of 1 to 4 equivalents relative to the copper complex compound of Formula 1.

18. The trifluoroalkylation method of claim 14, wherein the reaction of the trifluoroacetic acid with the ligand is performed in the presence of a reducing agent added in an amount of 1 to 5 equivalents relative to the ligand.

19. The trifluoroalkylation method of claim 14, wherein the reaction of the trifluoroacetic acid with the ligand is performed at 10 to 150°C for 5 to 120 minutes.

20. A method for producing a radiopharmaceutical having a ligand substituted with a [¹⁸F]fluoroalkyl group, comprising the method of any one of claims 1 to 19.

21. A building block composition for trifluoroalkylation comprising a copper complex compound represented by Formula 1 below: 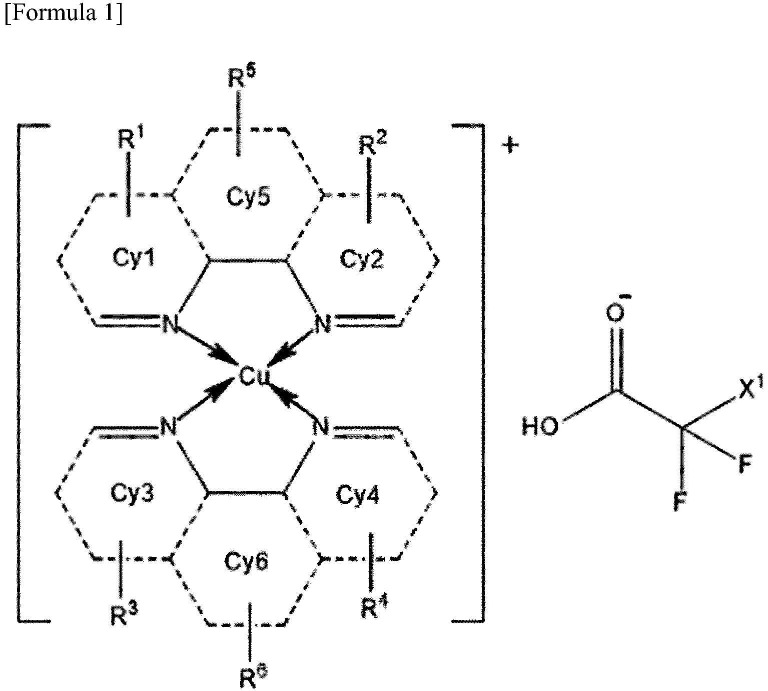 wherein
Cy1 to Cy4 are each independently a 4- to 8-membered heteroaryl or heterocycloalkenyl ring,
Cy5 and Cy6 are each independently a 0- to 8-membered cycloalkyl, cycloalkenyl, aryl, heteroaryl, heterocycloalkyl or heterocycloalkenyl ring,
R¹ to R⁶ are each independently hydrogen, a halogen, nitro, a C₁-C₆ alkyl group, or a C₆-C₁₄ aryl group, and are mono-substituents or the same or different multi-substituents, and
X¹ is a halogen other than a fluoro group.

22. The composition of claim 21, wherein the copper complex compound is a compound represented by Formula 6 or 7 below: 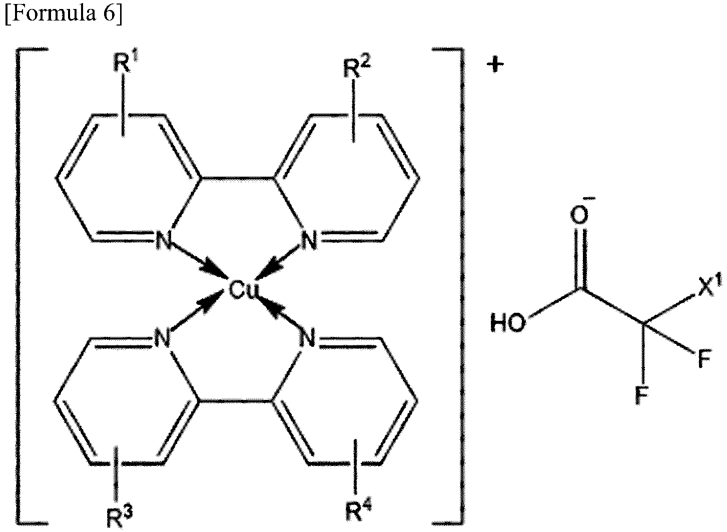 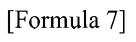 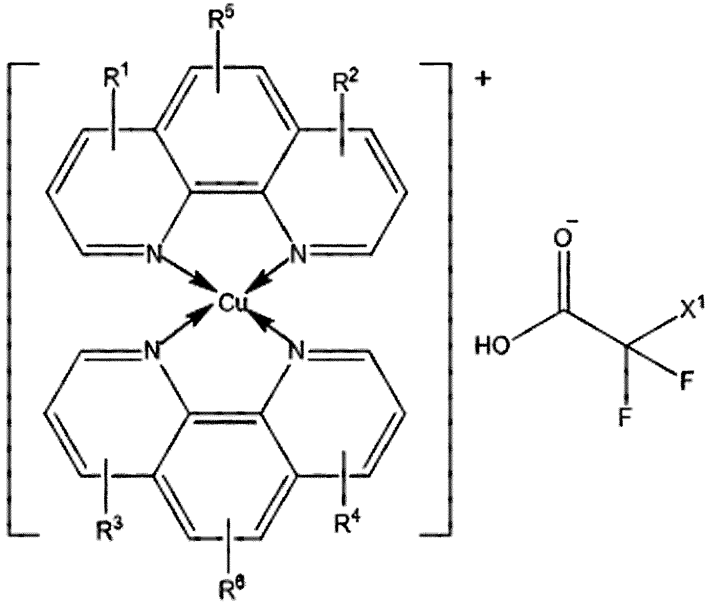 wherein R¹ to R⁶ and X¹ are as defined in claim 21.

23. A method of producing a trifluoroacetic acid represented by Formula 14 below by reacting a copper complex compound represented by Formula 1 below or a compound represented by Formula 2 below with [¹⁸F]fluoride ion or [¹⁹F]fluoride ion: 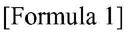 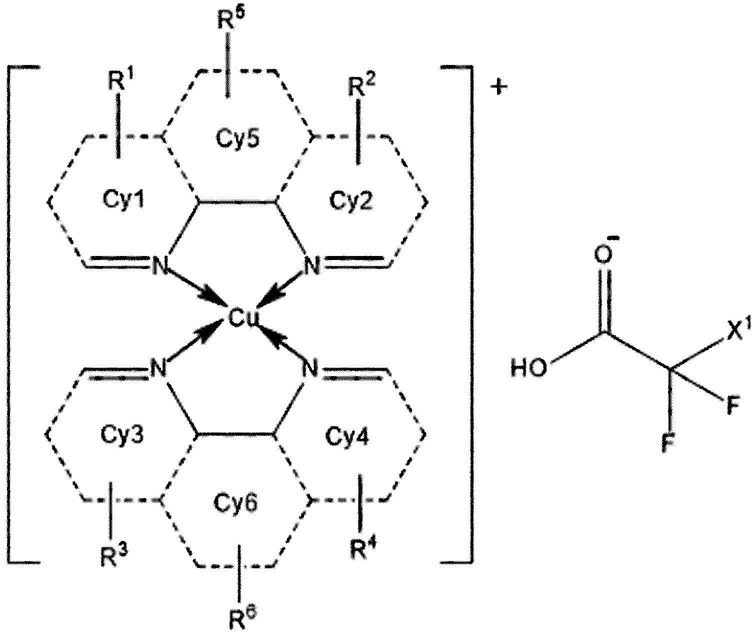 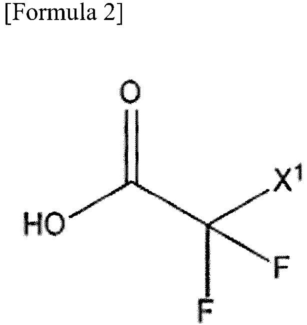 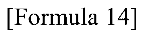 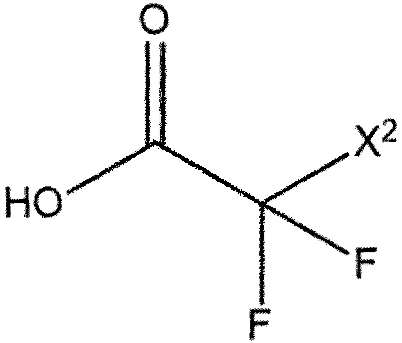 wherein
Cy1 to Cy4 are each independently a 4- to 8-membered heteroaryl or heterocycloalkenyl ring,
Cy5 and Cy6 are each independently a 0- to 8-membered cycloalkyl, cycloalkenyl, aryl, heteroaryl, heterocycloalkyl or heterocycloalkenyl ring,
R¹ to R⁶ are each independently hydrogen, a halogen, nitro, a C₁-C₆ alkyl group, or a C₆-C₁₄ aryl group, and are mono-substituents or the same or different multi-substituents, and
X¹ is a halogen other than a fluoro group, and
X² is ¹⁸F or ¹⁹F.
